# NEUE EUROPÄISCHE PATENTSCHRIFT

(11) **EP 1 716 106 B2**
(45) Veröffentlichungstag und Bekanntmachung des Hinweises auf die Entscheidung über den Einspruch: **08.01.2020**
(45) Hinweis auf die Patenterteilung: 09.03.2011
(21) Anmeldenummer: 05707030.2
(22) Anmeldetag: 27.01.2005
(51) Int. Cl.: C07C 253/10, C07C 255/07

(54) **HERSTELLUNG VON 3-PENTENNITRIL AUS 1,3-BUTADIEN**
PRODUCTION OF 3-PENTENENITRILE FROM 1,3-BUTADIENE
FABRICATION DE 3-PENTENE NITRILE A PARTIR DE 1,3-BUTADIENE

(30) Priorität: 29.01.2004 DE 102004004724
(43) Veröffentlichungstag der Anmeldung: 02.11.2006
(73) Patentinhaber: BASF SE, 67056 Ludwigshafen am Rhein (DE)
(72) Erfinder: SCHEIDEL, Jens, 69493 Hirschberg (DE); JUNGKAMP, Tim, B-2950 Kapellen (BE); BARTSCH, Michael, 67433 Neustadt (DE); HADERLEIN, Gerd, 67269 Grünstadt (DE); BAUMANN, Robert, 68159 Mannheim (DE); LUYKEN, Hermann, 67069 Ludwigshafen (DE)
(74) Vertreter: Féaux de Lacroix, Stefan
(86) Internationale Anmeldenummer: PCT/EP2005/000784
(87) Internationale Veröffentlichungsnummer: WO 2005/073175

(56) Entgegenhaltungen:
- WO-A-98/27054
- US-A- 4 277 314
- US-A- 4 434 316
- US-B1- 6 197 992
- US-B1- 6 337 429
- Decio Heringer Coutinho: "Synthesis and modification of mesoporous silica and the preparation of molecular sieve thin films via pulsed laser deposition", DlSSERTATION Presented to the Faculty of The University of Texas at Dallas in Partial Fulfillment of the Requirements for the Degree of DOCTOR OF
- John F Lane et al: "Allylic Rearrangement in the Reaction of Cuprous Cyanide with Butenyl Halides 1", Journal of the American Chemical Society, vol. 66, no. 4, 1 April 1944 (1944-04-01), pages 545-548,
- "Chapter 7 - Separation System" In: James M Douglas: "Conceptual Design of Chemical Processes", 1 January 1988 (1988-01-01), McGraw-Hill Book Company, New York ISBN: 0-07-017762-7 pages 163, 180-184,
- ARTHUR P. ET AL: "ADDITION OF HYDROGEN CYANIDE TO UNSATURATED COMPOUNDS", Journal of the American Chemical Society, vol. 76, 1 January 1954 (1954-01-01), pages 5364-5367, American Chemical Society, US
- McCabe & Smith's Unit Operations of Chemical Engineering, 3rd Edition, 1985, Chapter 18, pages 471-528
- Nylon 6,6 Supp B, September 1987, Process Economics Program Report No. 54B, Nylon 66, pages 201-213, 571-575
- Miller, Investigation of Selected Potential Environmental Contaminants: Butadiene and its Oligomenrs; Final Report, December 1978, pages 6 to 8
- Kister, Distillation Design, Chapter 3, 1992, pages 87 to 133

## Beschreibung

Die vorliegende Erfindung betrifft ein Verfahren zur Herstellung von 3-Pentennitril.

Adipodinitril ist ein wichtiges Ausgangsprodukt in der Nylonherstellung, das durch zweifache Hydrocyanierung von 1,3-Butadien erhalten wird. Dabei wird in einer ersten Hydrocyanierung 1,3-Butadien zu 3-Pentennitril hydrocyaniert, wobei als Nebenprodukte hauptsächlich 2-Methyl-3-butennitril, 4-Pentennitril, 2-Pentennitrile, 2-Methyl-2-butennitrile, C₉-Nitrile und Methylglutarnitril erhalten werden. In einer zweiten, sich anschließenden Hydrocyanierung wird 3-Pentennitril mit Cyanwasserstoff zu Adipodinitril umgesetzt. Beide Hydrocyanierungen werden durch Nickel(0)-Phosphor-Komplexe katalysiert.

Für die zweite Hydrocyanierung ist es wesentlich, dass das eingesetzte 3-Pentennitril frei von 2-Methyl-3-butennitril ist, da andernfalls 2-Methyl-3-butennitril zu dem unewünschten Nebenprodukt Methylglutarnitril hydrocyaniert würde.

Eine allgemeine Übersicht über die Nickel-katalysierte Olefinhydrocyanierung ist in Tolman et al., Adv. Cat. 33, 1 - 46 (1985) beschrieben.

Die Hydrocyanierung von 1,3-Butadien unter Verwendung eines Nickelkatalysators der Formel Ni[P(OR)₃]₄ wird in US 3,496,215 beschrieben. Nachteilig an diesem Verfahren ist, dass keine geeignete Technik zur vollständigen Rückgewinnung des 1,3-Butadiens oder des Katalysators angegeben ist.

US 5,693,843, US 5,696,280, US 5,821,378 und US 5,981,772 beschreiben Hydrocyanierungen von 1,3-Butadien mit multidentaten phosphorhaltigen Liganden, wobei jedoch in den einzelnen Ausführungsformen keine geeignete Verfahrensweise für die Rückgewinnung der Katalysatorkomponenten dargestellt ist.

Die Durchführung der Hydrocyanierung in einem oder mehreren Reaktoren und deren Verschaltung ist in US 4,810,815 beschrieben, wobei die Möglichkeit des kontinuierlichen Betriebs von Rührkesseln oder Kaskaden von Rührkesseln erwähnt wird, jedoch in Beispielen nur eine Semibatchfahrweise im Detail beschrieben ist, woraus für den Fachmann nicht direkt abgeleitet werden kann, unter welchen Bedingungen die Fahrweise in kontinuierlichen Rührkesseln zu erfolgen hat.

Ein Verfahren zur Abtrennung von organischen phosphorhaltigen Verbindungen und ihrer Metallkomplexe von organischen Nitrilen in der Hydrocyanierung von Olefinen wird in US 3,773,809 beschrieben. Die Abtrennung erfolgt dabei durch Inkontaktbringen des Produktes mit einem Cycloparaffin oder paraffinartigen Kohlenwasserstoff. Dabei bildet sich ein flüssiges mehrphasiges System. Diese Methode der Abtrennung und Rückgewinnung von Katalysatorkomponenten durch Extraktion ist wegen der zu geringen Konzentration von Dinitrilen im Reaktionsprodukt bei der Hydrocyanierung von 1,3-Butadien nicht anwendbar. WO - 98/27054 beschreibt Verfahren zur Herstellung monoolefinischen C₅-Mononitrile.

Darüber hinaus ist es für ein integriertes Verfahren zur Herstellung von 3-Pentennitril, bei dem sowohl der 1,3-Butadien als auch der Hydrocyanierungskatalysatorstrom zurückgeführt wird, entscheidend, dass das im molaren Überschuss gegenüber Cyanwasserstoff eingesetzte 1,3-Butadien effizient zurückgeführt wird.

Aufgabe der vorliegenden Erfindung ist es somit, ein integriertes Verfahren zur Herstellung von 3-Pentennitril durch Hydrocyanierung von 1,3-Butadien bereitzustellen, bei dem die Verfahrensausbeute bezüglich 1,3-Butadien möglichst hoch ist, obwohl sich das in handelsüblichem Butadien enthaltene, in der Hydrocyanierung unreaktive cis-2-Buten im Butadienkreislauf aufpegelt und daher ausgeschleust werden muss, was mit einer Zwangsausschleusung von 1,3-Butadien verbunden ist. Das erfindungsgemäße Verfahren wird demnach durch einen geringen Verlust an 1,3-Butadien infolge Ausschleusung gekennzeichnet.

Gelöst wird diese Aufgabe durch ein Verfahren zur Herstellung von 3-Pentennitril durch Hydrocyanierung von 1,3-Butadien, das durch die folgenden Verfahrensschritte gekennzeichnet ist:
(a) Umsetzung von 1,3-Butadien, das cis-2-Buten enthält, mit Cyanwasserstoff an mindestens einem Katalysator unter Erhalt eines Stromes 1, der 3-Pentennitril, 2-Methyl-3-butennitril, den mindestens einen Katalysator, 1,3-Butadien und Reste von noch nicht umgesetztem Cyanwasserstoff enthält,
(b) Destillation des Stromes 1 in einer Destillationsvorrichtung K1 unter Erhalt eines Stromes 2 als Kopfprodukt, der den überwiegenden Teil des 1,3-Butadiens aus Strom 1 enthält, und eines Stromes 3 als Sumpfprodukt, der 3-Pentennitril, den mindestens einen Katalysator, 2-Methyl-3-butennitril und den restlichen Teil des 1,3-Butadiens aus Strom 1 enthält, der nicht in Strom 2 abgetrennt wurde,
(c) Destillation des Stromes 3 in einer Destillationsvorrichtung K2 unter Erhalt eines Stromes 4 als Kopfprodukt, der 1,3-Butadien enthält, eines Stromes 5 an einem Seitenabzug der Kolonne, der 3-Pentennitril und 2-Methyl-3-butennitril enthält, und eines Stromes 6 als Sumpfprodukt, der den mindestens einen Katalysator enthält,
(d) Destillation des Stromes 5 unter Erhalt eines Stromes 7 als Kopfprodukt, der 2-Methyl-3-butennitril enthält, und eines Stromes 8 als Sumpfprodukt, der 3-Pentennitril enthält, wobei
die in Verfahrensschritt (b) verwendete Destillationsvorrichtung K1 mindestens eine Destillationskolonne mit einem Abtriebsteil umfasst und gegebenenfalls die in Verfahrensschritt (c) verwendete Destillationsvorrichtung K2 zwischen dem Zulauf des Stromes 3 und dem Abzug des Stromes 5 destillative Trennstufen aufweist und der Abzug des Stromes 5 in der Destillationsvorrichtung K2 tiefer als der Zulauf von Strom 3 angeordnet ist, und wobei
der in Verfahrensschritt (b) erhaltene Strom 2, der 1,3-Butadien enthält, in Verfahrensschritt (a) und der in Verfahrensschritt (c) erhaltene Strom 4, der 1,3-Butadien enthält, in Verfahrensschritt (a) und/oder (b) zurückgefahren werden, und ein Teilstrom 4b aus dem in Verfahrensschritt (c) erhaltenen Strom 4 ausgeschleust wird.

Der zuvor als überwiegender Teil des 1,3-Butadiens aus Strom 1 bezeichnete Anteil des 1,3-Butadiens aus Strom 1, der mit Strom 2 abgetrennt wird, bezieht sich auf einen Anteil von vorzugsweise mehr als 50 %, besonders bevorzugt mehr als 60 %, insbesondere mehr als 70 % des 1;3-Butadiens, das in Strom 1 enthalten ist. Das entsprechend verbleibende 1,3-Butadien aus Strom 1 wird über Strom 3 in den Verfahrensschritt (c) übergeführt.

Der ***Verfahrensschritt (a)*** umfasst die Umsetzung von 1,3-Butadien und Cyanwasserstoff an mindestens einem Katalysator. Als Katalysator werden homogen gelöste Nickel(0)-Katalysatoren-Komplexe verwendet.

Bei den Ni(0)-Komplexen, die phosphorhaltige Liganden und/oder freie phosphorhaltige Liganden enthalten, handelt es sich bevorzugt um homogen gelöste Nickel(0)-Komplexe.

Die phosphorhaltigen Liganden der Nickel(0)-Komplexe und die freien phosphorhaltigen Liganden sind vorzugsweise ausgewählt aus mono- oder bidentaten Phosphinen, Phosphiten, Phosphiniten und Phosphoniten.

Diese phosphorhaltigen Liganden weisen vorzugsweise die Formel I auf:

P (X¹R¹) (X²R²) (X³R³) (I)

Unter Verbindung I wird im Sinne der vorliegenden Erfindung eine einzelne Verbindung oder ein Gemisch verschiedener Verbindungen der vorgenannten Formel verstanden.

Erfindungsgemäß sind X¹, X², X³ unabhängig voneinander Sauerstoff oder Einzelbindung. Falls alle der Gruppen X¹, X² und X³ für Einzelbindungen stehen, so stellt Verbindung I ein Phosphin der Formel P(R¹R²R³) mit den für R¹, R² und R³ in dieser Beschreibung genannten Bedeutungen dar.

Falls zwei der Gruppen X¹, X² und X³ für Einzelbindungen stehen und eine für Sauerstoff, so stellt Verbindung I ein Phosphinit der Formel P(OR¹)(R²)(R³) oder P(R¹)(OR²)(R³) oder P(R¹)(R²)(OR³) mit den für R¹, R² und R³ weiter unten genannten Bedeutungen dar.

Falls eine der Gruppen X¹, X² und X³ für eine Einzelbindung steht und zwei für Sauerstoff, so stellt Verbindung I ein Phosphonit der Formel P(OR¹)(OR²)(R³) oder P(R¹)(OR²)(OR³) oder P(OR¹)(R²)(OR³) mit den für R¹, R² und R³ in dieser Beschreibung genannten Bedeutungen dar.

In einer bevorzugten Ausführungsform sollten alle der Gruppen X¹, X² und X³ für Sauerstoff stehen, so dass Verbindung I vorteilhaft ein Phosphit der Formel P(OR¹)(OR²)(OR³) mit den für R¹, R² und R³ weiter unten genannten Bedeutungen darstellt.

Erfindungsgemäß stehen R¹, R², R³ unabhängig voneinander für gleiche oder unterschiedliche organische Reste. Als R¹, R² und R³ kommen unabhängig voneinander Alkylreste, vorzugsweise mit 1 bis 10 Kohlenstoffatomen, wie Methyl, Ethyl, n-Propyl, i-Propyl, n-Butyl, i-Butyl, s-Butyl, t-Butyl, Aryl-Gruppen, wie Phenyl, o-Tolyl, m-Tolyl, p-Tolyl, 1-Naphthyl, 2-Naphthyl, oder Hydrocarbyl, vorzugsweise mit 1 bis 20 Kohlenstoffatomen, wie 1,1'-Biphenol, 1,1'-Binaphthol in Betracht. Die Gruppen R¹, R² und R³ können miteinander direkt, also nicht allein über das zentrale Phosphor-Atom, verbunden sein. Vorzugsweise sind die Gruppen R¹, R² und R³ nicht miteinander direkt verbunden.

In einer bevorzugten Ausführungsform kommen als Gruppen R¹, R² und R³ Reste ausgewählt aus der Gruppe bestehend aus Phenyl, o-Tolyl, m-Tolyl und p-Tolyl in Betracht. In einer besonders bevorzugten Ausführungsform sollten dabei maximal zwei der Gruppen R¹, R² und R³ Phenyl-Gruppen sein.

In einer anderen bevorzugten Ausführungsform sollten dabei maximal zwei der Gruppen R¹, R² und R³ o-Tolyl-Gruppen sein.

Als besonders bevorzugte Verbindungen I können solche der Formel I a

(o-Tolyl-O-)_{w} (m-Tolyl-O-)ₓ (p-Tolyl-O-)_{y} (Phenyl-O-)_{z} P (Ia)

eingesetzt werden, wobei w, x, y und z eine natürliche Zahl bedeuten, und folgende Bedingungen gelten: w + x + y + z = 3 und w, z ≤ 2.

Solche Verbindungen I a sind z.B. (p-Tolyl-O-)(Phenyl-O-)₂P, (m-Tolyl-O-)(Phenyl-O-)₂P, (o-Tolyl-O-) (Phenyl-O-)₂P, (p-Tolyl-O-)₂(Phenyl-O-)P, (m-Tolyl-O-)₂(Phenyl-O-)P, (o-Tolyl-O-)₂(Phenyl-O-)P, (m-Totyl-O-)(p-Tolyl-O)(Phenyl-O-)P, (o-Tolyl-O-)(p-Tolyl-O-)(Phenyl-O-)P, (o-Tolyl-O-)(m-Tolyl-O-)(Phenyl-O-)P, (p-Tolyl-O-)₃P, (m-Tolyl-O-)(p-Tolyl-O-)₂P, (o-Tolyl-O-)(p-Tolyl-O-)₂P, (m-Tolyl-O-)₂(p-Toluyl-O-)P, (o-Tolyl-O-)₂(p-Tolyl-O-)P, (o-Tolyl-O-)(m-Tolyl-O-)(p-Tolyl-O)P, (m-Tolyl-O-)₃P, (o-Tolyl-O-)(m-Tolyl-O-)₂P (o-Tolyl-O-)₂(m-Tolyl-O-)P, oder Gemische solcher Verbindungen.

Gemische enthaltend (m-Tolyl-O-)₃P, (m -Tolyl-O-)₂(p-Tolyl-O-)P, (m-Tolyl-O-)(p-Tolyl-O-)₂P und (p-Tolyl-O-)₃P kann man beispielsweise durch Umsetzung eines Gemisches enthaltend m-Kresol und p-Kresol, insbesondere im Molverhältnis 2:1, wie es bei der destillativen Aufarbeitung von Erdöl anfällt, mit einem Phosphortrihalogenid, wie Phosphortrichlorid, erhalten.

In einer anderen, ebenfalls bevorzugten Ausführungsform kommen als phosphorhaltige Liganden die in der DE-A 199 53 058 näher beschriebenen Phosphite der Formel I b in Betracht:

P (O-R¹)ₓ (O-R²)_{y} (O-R³)_{z} (O-R⁴)ₚ (Ib)

mit
- R¹:: aromatischer Rest mit einem C₁-C₁₈-Alkylsubstituenten in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromati- schen System verbindet, anellierten aromatischen System,
- R²:: aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoff- atom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der a- romatische Rest in o-Stellung zu dem Sauerstoffatom, das das Phosphoratom mit dem aromatischen System verbindet, ein Wasserstoffatom trägt,
- R³:: aromatischer Rest mit einem C₁-C₁₈-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aro- matische Rest in o-Stellung zu dem Sauerstoffatom, das das Phosphoratom mit dem aromatischen System verbindet, ein Wasserstoffatom trägt,
- R⁴:: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt,
- x :: 1 oder 2,
- y, z, p:: unabhängig voneinander 0, 1 oder 2 mit der Maßgabe, dass x+y+z+p = 3.

Bevorzugte Phosphite der Formel I b sind der DE-A 199 53 058 zu entnehmen. Als Rest R¹ kommen vorteilhaft o-Tolyl-, o-Ethyl-phenyl-, o-n-Propyl-phenyl-, o-Isopropyl-phenyl-, o-n-Butyl-phenyl-, o-sek-Butyl-phenyl-, o-tert-Butyl-phenyl-, (o-Phenyl)-Phenyl- oder 1-Naphthyl- Gruppen in Betracht.

Als Rest R² sind m-Tolyl-, m-Ethyl-phenyl-, m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen bevorzugt.

Als Rest R³ kommen vorteilhaft p-Tolyl-, p-Ethyl-phenyl-, p-n-Propyl-phenyl-, p-Isopropyl-phenyl-, p-n-Butyl-phenyl-, p-sek-Butyl-phenyl-, p-tert-Butyl-phenyl- oder (p-Phenyl)-Phenyl-Gruppen in Betracht.

Rest R⁴ ist bevorzugt Phenyl. Vorzugsweise ist p gleich null. Für die Indizes x, y, z und p in Verbindung I b ergeben sich folgende Möglichkeiten:

| x | y | z | p |
|---|---|---|---|
| 1 | 0 | 0 | 2 |
| 1 | 0 | 1 | 1 |
| 1 | 1 | 0 | 1 |
| 2 | 0 | 0 | 1 |
| 1 | 0 | 2 | 0 |
| 1 | 1 | 1 | 0 |
| 1 | 2 | 0 | 0 |
| 2 | 0 | 1 | 0 |
| 2 | 1 | 0 | 0 |

Bevorzugte Phosphite der Formel Ib sind solche, in denen p gleich null ist sowie R¹, R² und R³ unabhängig voneinander ausgewählt sind aus o-Isopropyl-phenyl, m-Tolyl und p-Tolyl, und R⁴ Phenyl ist.

Besonders bevorzugte Phosphite der Formel I b sind solche, in denen R¹ der o-Isopropyl-phenyl-Rest, R² der m-Tolylrest und R³ der p-Tolylrest ist mit den in der vorstehenden Tabelle genannten Indizes; außerdem solche, in denen R¹ der o-Tolylrest, R² der m-Tolylrest und R³ der p-Tolylrest ist mit den in der Tabelle genannten Indizes; weiterhin solche, in denen R¹ der 1-Naphthylrest, R² der m-Tolylrest und R³ der p-Tolylrest ist mit den in der Tabelle genannten Indizes; außerdem solche, in denen R¹ der o-Tolylrest, R² der 2-Naphthylrest und R³ der p-Tolylrest ist mit den in der Tabelle genannten Indizes; und schließlich solche, in denen R¹ der o-Isopropyl-phenyl-Rest, R² der 2-Naphthylrest und R³ der p-Tolylrest ist mit den in der Tabelle genannten Indizes; sowie Gemische dieser Phosphite.

Phosphite der Formel I b können erhalten werden, indem man
a) ein Phosphortrihalogenid mit einem Alkohol ausgewählt aus der Gruppe bestehend aus R¹OH, R²OH, R³OH und R⁴OH oder deren Gemische umsetzt unter Erhalt eines Dihalogenophosphorigsäuremonoesters,
b) den genannten Dihalogenophosphorigsäuremonoester mit einem Alkohol ausgewählt aus der Gruppe bestehend aus R¹OH, R²OH, R³OH und R⁴OH oder deren Gemische umsetzt unter Erhalt eines Monohalogenophosphorigsäurediesters und
c) den genannten Monohalogenophosphorigsäurediester mit einem Alkohol ausgewählt aus der Gruppe bestehend aus R¹OH, R²OH, R³OH und R⁴OH oder deren Gemische umsetzt unter Erhalt eines Phosphits der Formel I b.

Die Umsetzung kann in drei getrennten Schritten durchgeführt werden. Ebenso können zwei der drei Schritte kombiniert werden, also a) mit b) oder b) mit c). Alternativ können alle der Schritte a), b) und c) miteinander kombiniert werden.

Dabei kann man geeignete Parameter und Mengen der Alkohole ausgewählt aus der Gruppe bestehend aus R¹OH, R²OH, R³OH und R⁴OH oder deren Gemische durch einige einfache Vorversuche leicht ermitteln.

Als Phosphortrihalogenid kommen grundsätzlich alle Phosphortrihalogenide, vorzugsweise solche, in denen als Halogenid Cl, Br, I, insbesondere Cl, eingesetzt wird, sowie deren Gemische in Betracht. Es können auch Gemische verschiedener gleich oder unterschiedlich halogensubstituierter Phosphine als Phosphortrihalogenid eingesetzt werden. Besonders bevorzugt ist PCl₃. Weitere Einzelheiten zu den Reaktionsbedingungen bei der Herstellung der Phosphite I b und zur Aufarbeitung sind der DE-A 199 53 058 zu entnehmen.

Die Phosphite I b können auch in Form eines Gemisches verschiedener Phosphite I b als Ligand verwendet werden. Ein solches Gemisch kann beispielsweise bei der Herstellung der Phosphite I b anfallen.

Es ist allerdings bevorzugt, dass der phosphorhaltige Ligand mehrzähnig, insbesondere zweizähnig ist. Daher weist der verwendete Ligand vorzugsweise die Formel II 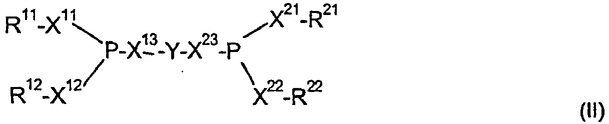 auf, worin bedeuten
- X¹¹, X¹², X¹³, X²¹, X²², X²³: unabhängig voneinander Sauerstoff oder Einzelbindung
- R¹¹, R¹²: unabhängig voneinander gleiche oder unterschiedliche, einzelne oder verbrückte organische Reste
- R²¹, R²²: unabhängig voneinander gleiche oder unterschiedliche, einzelne oder verbrückte organische Reste,
- Y: Brückengruppe

Unter Verbindung II wird im Sinne der vorliegenden Erfindung eine einzelne Verbindung oder ein Gemisch verschiedener Verbindungen der vorgenannten Formel verstanden.

In einer bevorzugten Ausführungsform können X¹¹, X¹², X¹³, X²¹, X²², X²³ Sauerstoff darstellen. In einem solchen Fall ist die Brückengruppe Y mit Phosphit-Gruppen verknüpft.

In einer anderen bevorzugten Ausführungsform können X¹¹ und X¹² Sauerstoff und X¹³ eine Einzelbindung oder X¹¹ und X¹³ Sauerstoff und X¹² eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²¹ und X²² Sauerstoff und X²³ eine Einzelbindung oder X²¹ und X²³ Sauerstoff und X²² eine Einzelbindung oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphonits, Phosphinits oder Phosphins, vorzugsweise eines Phosphonits, sein kann.

In einer anderen bevorzugten Ausführungsform können X¹³ Sauerstoff und X¹¹ und X¹² eine Einzelbindung oder X¹¹ Sauerstoff und X¹² und X¹³ eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphinits oder Phosphins, vorzugsweise eines Phosphinits, sein kann.

In einer anderen bevorzugten Ausführungsform können X¹¹, X¹² und X¹³ eine Einzelbindung darstellen, so dass das mit X¹¹, X¹² und X¹³ umgebene Phosphoratom Zentralatom eines Phosphins ist. In einem solchen Fall können X²¹, X²² und X²³ Sauerstoff oder X²¹, X²² und X²³ eine Einzelbindung darstellen, so dass das mit X²¹, X²² und X²³ umgebene Phosphoratom Zentralatom eines Phosphits oder Phosphins, vorzugsweise eines Phosphins, sein kann.

Als Brückengruppe Y kommen vorzugsweise substituierte, beispielsweise mit C₁-C₄-Alkyl, Halogen, wie Fluor, Chlor, Brom, halogeniertem Alkyl, wie Trifluormethyl, Aryl, wie Phenyl, oder unsubstituerte Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste R¹¹ und R¹² können unabhängig voneinander gleiche oder unterschiedliche organische Reste darstellen. Vorteilhaft kommen als Reste R¹¹ und R¹² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R²¹ und R²² können unabhängig voneinander gleiche oder unterscheidliche organische Reste darstellen. Vorteilhaft kommen als Reste R²¹ und R²² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R¹¹ und R¹² können einzeln oder verbrückt sein. Auch die Reste R²¹ und R²² können einzeln oder verbrückt sein. Die Reste R¹¹, R¹², R²¹ und R²² können alle einzeln, zwei verbrückt und zwei einzeln oder alle vier verbrückt sein in der beschriebenen Art.

In einer besonders bevorzugten Ausführungsform kommen die in US 5,723,641 genannten Verbindungen der Formel I, II, III, IV und V in Betracht. In einer besonders bevorzugten Ausführungsform kommen die in US 5,512,696 genannten Verbindungen der Formel I, II, III IV, V, VI und VII, insbesondere die dort in den Beispielen 1 bis 31 eingesetzten Verbindungen, in Betracht. In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzten Verbindungen, in Betracht.

In einer besonders bevorzugten Ausführungsform kommen die in US 5,512,695 genannten Verbindungen der Formel I, II, III, IV, V und VI, insbesondere die dort in den Beispielen 1 bis 6 eingesetzten Verbindungen, in Betracht. In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzten Verbindungen, in Betracht.

In einer besonders bevorzugten Ausführungsform kommen die in US 6,127,567 genannten Verbindungen und dort in den Beispielen 1 bis 29 eingesetzten Verbindungen in Betracht. In einer besonders bevorzugten Ausführungsform kommen die in US 6,020,516 genannten Verbindungen der Formel I, II, III, IV, V, VI, VII, VIII, IX und X, insbesondere die dort in den Beispielen 1 bis 33 eingesetzten Verbindungen, in Betracht. In einer besonders bevorzugten Ausführungsform kommen die in US 5,959,135 genannten Verbindungen und dort in den Beispielen 1 bis 13 eingesetzten Verbindungen in Betracht.

In einer besonders bevorzugten Ausführungsform kommen die in US 5,847,191 genannten Verbindungen der Formel I, II und III in Betracht. In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 13, 14, 15, 16, 17, 18, 19, 20 und 21 dargestellten Verbindungen, in Betracht. In einer besonders bevorzugten Ausführungsform kommen die in WO 01/14392 genannten Verbindungen, vorzugsweise die dort in Formel V, VI, VII, VIII, IX, X, XI, XII, XIII, XIV, XV, XVI, XVII, XXI, XXII, XXIII dargestellten Verbindungen, in Betracht.

In einer besonders bevorzugten Ausführungsform kommen die in WO 98/27054 genannten Verbindungen in Betracht. In einer besonders bevorzugten Ausführungsform kommen die in WO 99/13983 genannten Verbindungen in Betracht. In einer besonders bevorzugten Ausführungsform kommen die in WO 99/64155 genannten Verbindungen in Betracht.

In einer besonders bevorzugten Ausführungsform kommen die in der deutschen Patentanmeldung DE 100 380 37 genannten Verbindungen in Betracht. In einer besonders bevorzugten Ausführungsform kommen die in der deutschen Patentanmeldung DE 100 460 25 genannten Verbindungen in Betracht. In einer besonders bevorzugten Ausführungsform kommen die in der deutschen Patentanmeldung DE 101 502 85 genannten Verbindungen in Betracht.

In einer besonders bevorzugten Ausführungsform kommen die in der deutschen Patentanmeldung DE 101 502 86 genannten Verbindungen in Betracht. In einer besonders bevorzugten Ausführungsform kommen die in der deutschen Patentanmeldung DE 102 071 65 genannten Verbindungen in Betracht. In einer weiteren besonders bevorzugten Ausführungsform der vorliegenden Erfindung kommen die in der US 2003/0100442 A1 genannten phosphorhaltigen Chelatliganden in Betracht.

In einer weiteren besonders bevorzugten Ausführungsform der vorliegenden Erfindung kommen die in der nicht vorveröffentlichten deutschen Patentanmeldung Aktenzeichen DE 103 50 999.2 vom 30.10.2003 genannten phosphorhaltigen Chelatliganden in Betracht.

Die beschriebenen Verbindungen I, I a, I b und II sowie deren Herstellung sind an sich bekannt. Als phosphorhaltiger Ligand können auch Mischungen, enthaltend mindestens zwei der Verbindungen I, I a, I b und II, eingesetzt werden.

In einer besonders bevorzugten Ausführungsform des erfindungsgemäßen Verfahrens ist der phosphorhaltige Ligand des Nickel(0)-Komplexes und/oder der freie phosphorhaltige Ligand ausgewählt aus Tritolylphosphit, bidentaten phosphorhaltigen Chelatliganden, sowie den Phosphiten der Formel I b

P (O-R¹)ₓ (O-R²)_{y} (O-R³)_{z} (O-R⁴)ₚ (I b)

worin R¹, R² und R³ unabhängig voneinander ausgewählt sind aus o-Isopropyl-phenyl, m-Tolyl und p-Tolyl, R⁴ Phenyl ist; x gleich 1 oder 2 ist, und y, z, p unabhängig voneinander 0, 1 oder 2 sind mit der Maßgabe, dass x+y+z+p = 3 ist; und deren Mischungen.

Der Verfahrensschritt (a) des erfindungsgemäßen Verfahrens kann in jeder geeigneten, dem Fachmann bekannten Vorrichtung durchgeführt werden. Für die Reaktion kommen somit übliche Apparaturen in Betracht, wie sie beispielsweise in: Kirk-Othmer, Encyclopedia of Chemical Technology, 4. Ed., Vol. 20, John Wiley & Sons, New York, 1996, Seiten 1040 bis 1055 beschrieben sind, wie Rührkesselreaktoren, Schlaufenreaktoren, Gasumlaufreaktoren, Blasensäulenreaktoren oder Rohrreaktoren, jeweils gegebenenfalls mit Vorrichtungen zur Abfuhr von Reaktionswärme. Die Reaktion kann in mehreren, wie zwei oder drei, Apparaturen durchgeführt werden.

In einer bevorzugten Ausführungsform des erfindungsgemäßen Verfahrens haben sich Reaktoren mit Rückvermischungscharakteristik oder Kaskaden von Reaktoren mit Rückvermischungscharakteristik als vorteilhaft erwiesen. Als besonders vorteilhaft haben sich Kaskaden aus Reaktoren mit Rückvermischungscharakteristik erwiesen, die in Bezug auf die Dosierung von Cyanwasserstoff in Querstromfahrweise betrieben werden.

Die Hydrocyanierung kann in Gegenwart oder in Abwesenheit von einem Lösemittel durchgeführt werden. Wenn ein Lösemittel verwendet wird, so sollte das Lösemittel bei der gegebenen Reaktionstemperatur und dem gegebenen Reaktionsdruck flüssig und inert gegenüber den ungesättigten Verbindungen und dem mindestens einen Katalysator sein. Im Allgemeinen werden als Lösemittel Kohlenwasserstoffe, beispielsweise Benzol oder Xylol, oder Nitrile, beispielsweise Acetonitril oder Benzonitril, verwendet. Vorzugsweise wird allerdings ein Ligand als Lösemittel verwendet.

Die Reaktion kann in Batchfahrweise, kontinuierlich oder im Semibatchbetrieb durchgeführt werden.

Die Hydrocyanierungsreaktion kann durchgeführt werden, indem die Vorrichtung mit allen Reaktanten bestückt wird. Bevorzugt ist allerdings, wenn die Vorrichtung mit dem Katalysator, der ungesättigten organischen Verbindung und gegebenenfalls dem Lösemittel gefüllt wird. Vorzugsweise schwebt der gasförmige Cyanwasserstoff über der Oberfläche der Reaktionsmischung oder wird durch die Reaktionsmischung geleitet. Eine weitere Verfahrensweise zum Bestücken der Vorrichtung ist das Befüllen der Vorrichtung mit dem Katalysator, Cyanwasserstoff und gegebenenfalls dem Lösemittel und das langsame Zuspeisen der ungesättigten Verbindung zu der Reaktionsmischung. Alternativ ist auch möglich, dass die Reaktanten in den Reaktor eingeführt werden und die Reaktionsmischung auf die Reaktionstemperatur gebracht wird, bei welcher der Cyanwasserstoff flüssig zu der Mischung gegeben wird. Darüber hinaus kann der Cyanwasserstoff auch vor dem Erwärmen auf Reaktionstemperatur zugegeben werden. Die Reaktion wird unter konventionellen Hydrocyanierungsbedingungen für Temperatur, Atmosphäre, Reaktionszeit, etc. durchgeführt.

Vorzugsweise wird die Hydrocyanierung kontinuierlich in einem oder mehreren gerührten Verfahrensschritten durchgeführt. Wenn eine Mehrzahl von Verfahrensschritten verwendet wird, so ist es bevorzugt, dass die Verfahrensschritte in Serie geschaltet sind. Dabei wird das Produkt von einem Verfahrensschritt direkt in den nächsten Verfahrensschritt überführt. Der Cyanwasserstoff kann direkt in den ersten Verfahrensschritt oder zwischen den einzelnen Verfahrensschritten zugeführt werden.

Wenn das erfindungsgemäße Verfahren im Semibatchbetrieb durchgeführt wird, so ist es bevorzugt, dass im Reaktor die Katalysatorkomponenten und 1,3-Butadien vorgelegt werden, während Cyanwasserstoff über die Reaktionszeit hinweg in die Reaktionsmischung dosiert wird.

Die Reaktion wird vorzugsweise bei absoluten Drücken von 0,1 bis 500 MPa, besonders bevorzugt 0,5 bis 50 MPa, insbesondere 1 bis 5 MPa, durchgeführt. Die Reaktion wird vorzugsweise bei Temperaturen von 273 bis 473 K, besonders bevorzugt 313 bis 423 K, insbesondere bei 333 bis 393 K, durchgeführt. Dabei haben sich durchschnittliche mittlere Verweilzeiten der flüssigen Reaktorphase im Bereich von 0,001 bis 100 Stunden, vorzugsweise 0,05 bis 20 Stunden, besonders bevorzugt 0,1 bis 5 Stunden, jeweils pro Reaktor, als vorteilhaft erwiesen.

Die Reaktion kann in einer Ausführungsform in flüssiger Phase in Gegenwart einer Gasphase und gegebenenfalls einer festen suspendierten Phase ausgeführt werden.

Dabei können die Ausgangsstoffe Cyanwasserstoff und 1,3-Butadien jeweils flüssig oder gasförmig zudosiert werden.

Die Reaktion kann in einer weiteren Ausführungsform in flüssiger Phase durchgeführt werden, wobei der Druck im Reaktor so bemessen ist, dass alle Einsatzstoffe wie 1,3-Butadien, Cyanwasserstoff und der mindestens eine Katalysator flüssig zudosiert werden und in der Reaktionsmischung in flüssiger Phase vorliegen. Dabei kann eine feste suspendierte Phase im Reaktionsgemisch vorliegen, die auch zusammen mit dem mindestens einen Katalysator zudosiert werden kann, beispielsweise bestehend aus Abbauprodukten des Katalysatorsystems, enthaltend unter anderem Nickel(II)-Verbindungen.

Im Verfahrensschritt (a) wird ein Strom 1, der 3-Pentennitril, 2-Methyl-3-butennitril, den mindestens einen Katalysator und nicht umgesetztes 1,3-Butadien sowie Reste von nicht umgesetztem Cyanwasserstoff enthält, erhalten. Dieser Strom 1 weist vorzugsweise die folgende Zusammensetzung auf: 1 bis 80 Gew.-%, besonders bevorzugt 5 bis 50 Gew.-%, des mindestens einen Katalysators, 0,1 bis 50 Gew.-%, besonders bevorzugt 1 bis 25 Gew.-%, 1,3-Butadien, 1 bis 80 Gew.-%, besonders bevorzugt 10 bis 50 Gew.-%, Pentennitrile, umfassend trans-3-Pentennitril, 2-Methyl-3-butennitril sowie weitere Pentennitrilisomere und 0,1 Gew.-ppm bis 10 Gew.-%, besonders bevorzugt 10 Gew.-ppm bis 1 Gew.-%, Cyanwasserstoff jeweils bezogen auf die Gesamtmasse des Stromes 1.

Der Strom 1, der 3-Pentennitril, 2-Methyl-3-butennitril, den mindestens einen Katalysator und nicht umgesetztes 1,3-Butadien enthält, wird anschließend in ***Verfahrensschritt (b)*** in eine Destillationsvorrichtung K1 überführt. In dieser Destillationsvorrichtung erfolgt eine Destillation des Stromes 1 unter Erhalt eines an 1,3-Butadien reichen Stromes 2 als Kopfprodukt und eines an 1,3-Butadien armen Stromes 3 als Sumpfprodukt, der 3-Pentennitril, den mindestens einen Katalysator und 2-Methyl-3-butennitril enthält.

Der Verfahrensschritt (b) des erfindungsgemäßen Verfahrens kann in jeder geeigneten, dem Fachmann bekannten Vorrichtung durchgeführt werden. Für die Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen oder einstufige Verdampfer, wie Fallfilmverdampfer, Dünnschichtverdampfer, Flashverdampfer, Mehrphasenwendelrohrverdampfer, Naturumlaufverdampfer oder Zwangsumlaufentspannungsverdampfer. Die Destillation kann in mehreren, wie zwei oder drei Apparaturen, vorzugsweise in einer einzigen Apparatur durchgeführt werden.

In einer bevorzugten Ausführungsform des erfindungsgemäßen Verfahrens sind in der Destillationsvorrichtung Kolonneneinbauten mit strukturierter Packung vorhanden, die vorzugsweise zwischen 2 und 60, besonders bevorzugt zwischen 3 und 40, insbesondere zwischen 4 und 20, Trennstufen erzeugen.

In einer besonders bevorzugten Ausführungsform des erfindungsgemäßen Verfahrens wird die mindestens eine zur Destillationsvorrichtung von Verfahrensschritt (b) gehörige Verdampferstufe so ausgeführt, dass das zu verdampfende Material möglichst wenig thermische Schädigung erleidet, wie es beispielsweise durch Fallfilmverdampfer, Mehrphasenwendelrohrverdampfer, Dünnschichtverdampfer oder Kurzwegverdampfer durch kurze Kontaktzeiten des Materials an der Verdampferoberfläche und möglichst geringe Temperaturen der Verdampferoberflächen erreicht wird.

In einer bevorzugten Ausführungsform des erfindungsgemäßen Verfahrens wird die Destillationsvorrichtung von Verfahrensschritt (b) mit einem geteilten Sumpf betrieben, wobei man aus einem ersten Sumpf der betreffenden Destillationskolonne einen im Verhältnis zum Strom 3 im Allgemeinen um ein Vielfaches größeren Umlaufstrom zum Verdampfer fährt, den flüssigen Ablaufstrom aus dem Verdampfer jedoch nicht direkt in den ersten Sumpf zurückgibt, sondern in einen zweiten Sumpf, der vom ersten Sumpf getrennt ist, auffängt, aus dem zweiten Sumpf den Strom 3 erhält und den verbleibenden Überschuss vom Verdampferumlaufstrom in den ersten Sumpf überlaufen lässt, wobei als Strom 3 aus dem zweiten Sumpf eine Mischung erhalten wird, die gegenüber dem aus dem ersten Sumpf abgezogenen Verdampferumlaufstrom an Leichtsiedern abgereichert ist. Als Verdampfer wird dabei vorzugsweise ein Fallfilmverdampfer verwendet.

In einer weiteren bevorzugten Ausführungsform des erfindungsgemäßen Verfahrens wird die Destillation bei mittleren Verweilzeiten der flüssigen Phase im Sumpfbereich der ein oder mehreren Destillationsapparaturen in dem Verfahrensschritt (b) von zusammen weniger als 10 Stunden, besonders bevorzugt weniger als 5 Stunden, insbesondere weniger als 1 Stunde, durchgeführt .

In einer weiteren bevorzugten Ausführungsform des erfindungsgemäßen Verfahrens wird die Kondensation am Kopf der Destillationsvorrichtung so durchgeführt, dass ein Teilstrom vom Kopfaustrag in den Kondensator zurückgespült wird.

Die Destillation kann in einer weiteren bevorzugten Ausführungsform des erfindungsgemäßen Verfahrens mit einem Direktkondensator ausgeführt werden, so dass die Kondensation in einem Kolonnenschuss durchgeführt wird, der vorzugsweise ausgestattet ist mit einer strukturierten Kolonnenpackung, einer Fangtasse unterhalb dieser Packung, einem flüssigen Abzug aus der Fangtasse, einem an den flüssigen Abzug angeschlossenen Umpumpkreislauf mit Pumpe und Wärmetauscher sowie mindestens einer Vorrichtung zur Aufgabe des umgepumpten Flüssigstroms auf die Packung oberhalb der Fangtasse.

Die in Verfahrensschritt (b) verwendete Destillationsvorrichtung K1 umfasst eine Destillationskolonne mit Abtriebsteil, wobei die Destillationskolonne vorzugsweise 2 bis 60, besonders bevorzugt 3 bis 40, insbesondere 4 bis 20, theoretische Trennstufen aufweist.

Um eine möglichst hohe Verfahrensausbeute bezüglich 1,3-Butadien trotz der nur teilweise erfolgten Umsetzung in Schritt (a) zu erreichen, wird der an 1,3-Butadien reiche Strom 2 in den Verfahrensschritt (a) zurückgeführt.

In einer weiteren Ausführungsform kann bei der Destillation des Schrittes (b) das für die Umsetzung in Verfahrensschritt (a) zusätzlich benötigte 1,3-Butadien in den Kopfbereich der Kolonne oder in den Strom 2 zugefügt werden.

In einer weiteren Ausführungsform enthält das zugefügte 1,3-Butadien einen Stabilisator, wie tert.-Butylbrenzkatechin oder 2,6-Di-tert.-butyl-para-kresol, gemäß Beschreibung in "Ullmann's Encyclopedia Of Industrial Chemistry, 6th Edition, 2000 Electronic Release, Kapitel "Butadiene - 6. Stabilization, Storage and Transportation".

In einer besonders bevorzugten Ausführungsform des erfindungsgemäßen Verfahrens wird das entweder direkt im Verfahrensschritt (a) eingesetzte oder zu Verfahrensschritt (b) zugefügte und über Strom 2 in Schritt (a) überführte 1,3-Butadien durch Kontaktieren mit Molsieb mit einer Porengröße kleiner 10 Ångström oder durch Kontaktieren mit Aluminiumoxid von Wasser und gegebenenfalls dem Stabilisator befreit.

In einer weiteren besonders bevorzugten Ausführungsform weist das im Verfahren verwendete, d.h. das direkt in Verfahrensschritt (a) eingesetzte oder das in den Strom 2 zugeführte 1,3-Butadien keinen Stabilisator auf, wobei durch eine geeignete Wahl der Druckverhältnisse die Kondensationstemperaturen im Kopfbereich der Destillationseinrichtung von Verfahrensschritt (b) kleiner 293 K gehalten werden, um eine Polymerisation von 1,3-Butadien zu verhindern, insbesondere um das Wachstum von Popcorn-Polymerkeimen zu begrenzen.

Im handelsüblichen 1,3-Butadien ist cis-2-Buten in nennenswerten Mengen enthalten.

1-Buten entsteht als Nebenprodukt der Hydrocyanierung von 1,3-Butadien mit Nickel(0)-Katalysatoren.

Sowohl cis-2-Buten als auch 1-Buten pegeln sich im Kreislauf des 1,3-Butadiens des erfindungsgemäßen Verfahrens auf, je nachdem, wie gut die Effizienz der Rückführung ist. Je vollständiger 1,3-Butadien zurückgeführt wird, desto eher machen sich die Aufpegelungen bemerkbar.

Der Strom 2 wird somit vorzugsweise so erzeugt, dass er weniger als 50 Gew.-%, besonders bevorzugt weniger als 25 Gew.-%, insbesondere weniger als 15 Gew.-%, und vorzugsweise mehr als 1 Gew.-%, besonders bevorzugt mehr als 2,5 Gew.-%, insbesondere mehr als 5 Gew.-%, in Summe trans-2-Buten, cis-2-Buten und 1-Buten enthält. Der Rest ist im Wesentlichen 1,3-Butadien.

Eine Möglichkeit zur Abtrennung von cis-2-Buten aus dem Butadienkreislauf besteht erfindungsgemäß darin, die Destillationsvorrichtung K1 so zu betreiben, dass unterhalb des Zulaufs von Strom 1 Trennstufen wirksam sind, die eine Anreicherung von cis-2-Buten gegenüber 1,3-Butadien in Strom 3 zulassen. Es erfolgt eine Ausschleusung in Verfahrenschritt (c) in Form des Stromes 4b, der wie nachfolgend beschrieben aus Strom 3 erzeugt wird.

Die Ausschleusungen erfolgen vorzugsweise gasförmig.

Der absolute Druck in Verfahrensschritt (b) beträgt vorzugsweise 0,001 bis 100 bar, besonders bevorzugt 0,01 bis 10 bar, insbesondere 0,5 bis 5 bar. Die Destillation wird so durchgeführt, dass die Temperatur im Sumpf der Destillationsvorrichtung vorzugsweise 30 bis 140 °C, besonders bevorzugt 50 bis 130 °C, insbesondere 60 bis 120 °C, beträgt. Die Destillation wird so durchgeführt, dass die Kondensationstemperatur am Kopf der Destillationsvorrichtung vorzugsweise -50 bis 140 °C, besonders bevorzugt - 15 bis 60 °C, insbesondere 5 bis 45 °C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten

Das Rücklaufverhältnis am Kopf der Destillationsvorrichtung wird vorzugsweise so eingestellt, dass der Strom 2 1 bis 1000 ppm, besonders bevorzugt 5 bis 500 ppm, insbesondere 10 bis 200 ppm, 2 Methyl-3-butennitril enthält.

In Verfahrensschritt (b) wird ein an 1,3-Butadien reicher Strom 2 als Kopfprodukt und ein an 1,3-Butadien armer Strom 3 als Sumpfprodukt erhalten. Die Bezeichnung der Ströme als an 1,3-Butadien reich bzw. arm bezieht sich dabei auf den Gehalt an 1,3-Butadien des in Verfahrensschritt (b) eingesetzten Stromes 1.

In einer bevorzugten Ausführungsform des erfindungsgemäßen Verfahrens enthält der an 1,3-Butadien reiche Strom 2 in Summe 50 bis 100 Gew.-%, besonders bevorzugt 80 bis 100 Gew.-%, insbesondere 85 bis 99 Gew.-%, 1,3-Butadien und Buten-Isomere sowie in Summe 0 bis 50 Gew.-%, besonders bevorzugt 0 bis 20 Gew.-%, insbesondere 10 Gew.-ppm bis 1 Gew.-%, Pentennitril-Isomere, von denen im Wesentlichen 2-Methyl-3-butennitril und trans-3-Pentennitril im Strom 2 vertreten sind.

In einer bevorzugten Ausführungsform des erfindungsgemäßen Verfahrens enthält der an 1,3-Butadien arme Strom 3 in Summe 0 bis 50 Gew.-%, besonders bevorzugt 1 bis 30 Gew.-%, insbesondere 2 bis 20 Gew.-%, 1,3-Butadien und Buten-Isomere bezogen auf die Gesamtmasse des Stromes 3. In einer besonders bevorzugten Ausführungsform des erfindungsgemäßen Verfahrens werden die zuvor genannten Spezifikationen an 1,3-Butadien sowohl im Strom 2 als auch im Strom 3 erreicht.

Der in Verfahrensschritt (b) erhaltene Strom 2, der 1,3-Butadien enthält, wird, vor seiner Rückführung in Verfahrensschritt (a), vorzugsweise kondensiert. Dieses kann beispielsweise an einem Kondensator durch indirekte Wärmeabfuhr erfolgen.

Alternativ ist es auch möglich, dass in Verfahrensschritt (b) im Verstärkungsteil der Destillationskolonne ein Strom an einem Seitenabzug der Destillationsvorrichtung K1 im Siedezustand erhalten wird, an einem Kondensator durch indirekte Wärmeabfuhr unter Erhalt eines unterkühlten Stromes kondensiert und auf den Kopf der Destillationsvorrichtung K1 zurückgeführt wird, wobei vor oder nach der Kondensation ein Strom 2' aus dem Strom abgezogen und der Strom 2' anstelle des Stromes 2 in Verfahrensschritt (a) zurückgeführt wird.

Dabei ist es bevorzugt, dass dem Strom 2' kein Stabilisator zugesetzt wird. Der erhaltene Strom 2' kann zum Zwecke seines wirtschaftlichen Einsatzes in den Verfahrensschritt (a) zurückgeführt werden.

Der Strom 2' ist von seiner Verwendung als gleichwertig zu Strom 2 zu betrachten. Aussagen zu Strom 2 sind deshalb ebenso für Strom 2' gültig und umgekehrt.

Der aus Verfahrensschritt (b) stammende an 1,3-Butadien arme Strom 3, der 3-Pentennitril, den mindestens einen Katalysator und 2-Methyl-3-butennitril enthält, wird anschließend in ***Verfahrensschritt (c)*** in eine Destillationsvorrichtung überführt. In dieser Destillationsvorrichtung erfolgt eine Destillation des Stromes 3 unter Erhalt eines Stromes 4 als Kopfprodukt, der 1,3-Butadien enthält, eines Stromes 5 an einem Seitenabzug der Kolonne, der 3-Pentennitril und 2-Methyl-3-butennitril enthält, und eines Stromes 6 als Sumpfprodukt, der den mindestens einen Katalysator enthält.

Der Verfahrensschritt (c) des erfindungsgemäßen Verfahrens kann in jeder geeigneten, dem Fachmann bekannten Vorrichtung durchgeführt werden. Für diese Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen oder einstufige Verdampfer, wie Fallfilmverdampfer, Dünnschichtverdampfer, Flashverdampfer, Mehrphasenwendelrohrverdampfer, Naturumlaufverdampfer oder Zwangsumlaufentspannungsverdampfer. Die Destillation kann in mehreren, wie zwei oder drei Apparaturen, vorzugsweise in einer Apparatur durchgeführt werden.

In einer besonders bevorzugten Ausführungsform wird als Destillationsvorrichtung in Verfahrensschritt (c) mindestens eine Destillationskolonne ausgewählt, die einen Abtriebsteil umfasst, besonders bevorzugt nur eine Destillationskolonne, die nur einen Abtriebsteil aufweist.

Die Destillationsvorrichtung ist vorzugsweise mit einer strukturierten Packung ausgestattet, die 2 bis 50, besonders bevorzugt 3 bis 40, insbesondere 4 bis 30, theoretische Trennstufen erzeugt.

In einer besonders bevorzugten Ausführungsform des erfindungsgemäßen Verfahrens werden die mindestens eine zur Destillationsvorrichtung von Verfahrensschritt (c) gehörigen Verdampferstufen so ausgeführt, dass das zu verdampfende Material möglichst wenig thermische Schädigung erleidet, wie es beispielsweise durch Fallfilmverdampfer, Mehrphasenwendelrohrverdampfer, Dünnschichtverdampfer oder Kurzwegverdampfer, durch kurze Kontaktzeiten des Materials an der Verdampferoberfläche und möglichst geringe Temperaturen der Verdampferoberflächen erreicht wird.

In einer weiteren bevorzugten Ausführungsform des erfindungsgemäßen Verfahrens wird die Destillation bei mittleren Verweilzeiten der flüssigen Phase im Sumpfbereich der Destillationsapparaturen in dem Verfahrensschritt (c) von zusammen weniger als 10 Stunden, besonders bevorzugt weniger als 5 Stunden, insbesondere weniger als 1 Stunde, durchgeführt.

In einer besonders bevorzugten Ausführungsform des erfindungsgemäßen Verfahrens wird die Destillation bei mittleren Verweilzeiten der flüssigen Phase im Sumpfbereich der Destillationsapparaturen in den Verfahrensschritten (b) und (c) von zusammen weniger als 10 Stunden, besonders bevorzugt weniger als 5 Stunden, insbesondere weniger als 1 Stunde, durchgeführt.

Der absolute Druck in Verfahrensschritt (c) beträgt vorzugsweise 0,001 bis 10 bar, besonders bevorzugt 0,010 bis 1 bar, insbesondere 0,020 bis 0,5 bar. Die Destillation wird so durchgeführt, dass die Temperatur im Sumpf der Destillationsvorrichtung vorzugsweise 30 bis 140 °C, besonders bevorzugt 40 bis 130°C, insbesondere 50 bis 120°C, beträgt. Die Destillation wird so durchgeführt, dass die Kondensationstemperatur am Kopf der Destillationsvorrichtung vorzugsweise -20 bis 140 °C, besonders bevorzugt -10 bis 80°C, insbesondere -5 bis 60 °C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten.

In der Destillation des Verfahrensschrittes (c) wird ein Strom 4 als Kopfprodukt erhalten. Dieser Strom 4 enthält vorzugsweise in Summe 50 bis 100 Gew.-%, besonders bevorzugt 80 bis 100 Gew.-%, insbesondere 90 bis 99,9 Gew.-%, 1,3-Butadien und Buten-Isomere sowie in Summe 0 bis 50 Gew.-%, besonders bevorzugt 0 bis 20 Gew.-%, insbesondere 10 Gew.-ppm bis 10 Gew.-%, Pentennitril-Isomere, von denen im Wesentlichen 2-Methyl-3-butennitril und trans-3-Pentennitril im Strom 4 vertreten sind.

In einer bevorzugten Ausführungsform des erfindungsgemäßen Verfahrens wird der Strom 4 gasförmig in mindestens einem Kondensator am Kopf der Destillationsvorrichtung erhalten, wobei in dem mindestens einen Kondensator Pentennitril-Komponenten aus dem Brüdenstrom der Destillationsvorrichtung von Verfahrensschritt (c) im oben genannten Bereich von Kondensationsbedingungen wie Druck und Temperatur zumindest teilweise auskondensiert werden und in die Kolonne zumindest teilweise flüssig als Pentennitrile sowie 1,3-Butadien und Buten-Isomere enthaltender Strom zurückgeführt werden.

Um die Verfahrensausbeute an eingesetztem 1,3-Butadien in dem erfindungsgemäßen Verfahren zu erhöhen, wird der Strom 4 in den Verfahrensschritt (a) direkt oder indirekt zurückgeführt. Unter einer indirekten Rückführung des Stromes 4 in den Verfahrensschritt (a) wird dabei verstanden, dass der Strom 4 zunächst in die Destillationsvorrichtung K1 von Verfahrenschritt (b) und dann über den Strom 2 in den Verfahrensschritt (a) zurückgefahren wird.

Die indirekte Rückführung des Stromes 4 ist dabei besonders bevorzugt, wobei die Pentennitril-Komponenten, die je nach Destillationsbedingungen in Strom 4 enthalten sein können, durch Zurückführen des Stromes 4 in die Destillationsvorrichtung von Verfahrensschritt (b) vorzugsweise aus dem Strom 4 abgetrennt werden und letztendlich nur der 1,3-Butadien- und Buten-Isomeren-Anteil von Strom 4 über Strom 2 in Schritt (a) zurückgeführt wird.
Dabei kann der Strom 4 vor seiner Rückführung zusätzlich einer oder mehreren verfahrenstechnischen Aufarbeitungen, beispielsweise einer Verdichtung auf einen höheren Druck, unterzogen werden.

Der Strom 4 wird in einer Ausführungsform des erfindungsgemäßen Verfahrens ohne oder nach Verzögerung in die Destillationsvorrichtung K1 von Verfahrensschritt (b) teilweise zurückgefahren (Strom 4a). Aus dem Strom 4 wird einen Teilstrom 4b flüssig oder gasförmig zur Ausschleusung entnommen. Dies ist besonders vorteilhaft, weil in Strom 4 ein höherer Anteil an Buten-Isomeren enthalten ist und somit weniger Butadien als in Strom 2, die Zwangsausschleusung an Butadien somit geringer und die Verfahrensausbeute somit höher wird, wobei der Gehalt an Buten-Isomeren auf dem zuvor als vorteilhaft beschriebenen Niveau gehalten werden kann.

Der Gehalt an trans-2-Buten, cis-2-Buten und 1-Buten in Summe in dem rückgeführten Strom 4 bzw. 4a beträgt vorzugsweise mehr als 2 Gew.-%, besonders bevorzugt mehr als 10 Gew.-%, insbesondere mehr als 15 Gew.-%, und vorzugsweise weniger als 80 Gew.-%, besonders bevorzugt weniger als 70 Gew.-%, insbesondere weniger als 50 Gew.-%.

Vor Erhalt von Strom 4 werden nitrilhaltige Verbindungen vorzugsweise durch mehrstufige Kondensationen des Brüdenstroms der Destillationsvorrichtung K2 abgereichert. Der Strom 4 oder 4a, der an der Destillationsvorrichtung K2 in Verfahrensschritt (c) erhalten wird, wird vorzugsweise dampfförmig abgezogen und mit einer Kompressionsvorrichtung V1 und unter Druckerhöhung verdichtet. Dabei wird ein verdichteter Strom 4 oder 4a erhalten.

Dieser verdichtete Strom 4 oder 4a wird vorzugsweise durch Kondensation verflüssigt. Dabei bildet sich ein verflüssigter Strom 4 oder 4a.

Der so verdichtete und/oder verflüssigte Strom 4 wird anschließend vorzugsweise in die Destillationsvorrichtung K1 von Verfahrensschritt (b) zurückgeführt.

In einer besonders bevorzugten Ausführungsform wird der Strom 4 oder 4a in den Rücklaufteil des geteilten Sumpfes der Destillationsvorrichtung in Verfahrensschritt (b) eingeleitet.

Der Strom 4a ist von seiner Verwendung als gleichwertig zu Strom 4 zu betrachten. Aussagen zu Strom 4 sind deshalb ebenso für Strom 4a gültig und umgekehrt.
In dem Verfahrensschritt (c) wird neben dem Strom 4 ein weiterer Strom 5 erhalten, der an einem Seitenabzug der Kolonne gewonnen wird. Dieser Strom 5 enthält 3-Pentennitril und 2-Methyl-3-butennitril neben anderen Pentennitril-Isomeren und Restbestandteilen an 1,3-Butadien und Buten-Isomeren. Der Anteil an 3-Pentennitril und 2-Methyl-3-butennitril in dem Strom 5 beträgt in Summe vorzugsweise 80 bis 100 Gew.-%, besonders bevorzugt 85 bis 99,998 Gew.-%, insbesondere 90 bis 99,9 Gew.-%, jeweils bezogen auf den Strom 5. Der Anteil an 1,3-Butadien und Buten-Isomeren in dem Strom 5 beträgt vorzugsweise 0 bis 20 Gew.-%, besonders bevorzugt 10 Gew.-ppm bis 5 Gew.-%, insbesondere 50 Gew.-ppm bis 2 Gew.-%, jeweils bezogen auf den Strom 5. Der Strom 5 wird vorzugsweise dampfförmig entnommen.

Der Seitenabzug der Destillationsvorrichtung befindet sich vorzugsweise unterhalb der Zulaufstelle von Strom 3, besonders bevorzugt in einer Position entsprechend 1 bis 20, insbesondere 2 bis 10, destillativen Trennstufen unterhalb der Zulaufstelle von Strom 3.

Als Sumpfprodukt erhält man im Verfahrensschritt (c) einen Strom 6, der den mindestens einen Katalysator sowie trans-3-Pentennitril und 2-Methyl-3-butennitril enthält. Der Anteil an Pentennitril-Isomeren in dem Strom 6 beträgt in Summe vorzugsweise 0,1 bis 80 Gew.-%, besonders bevorzugt 5 bis 50 Gew.-%, insbesondere 10 bis 40 Gew.-%, jeweils bezogen auf den Strom 6.

Darüber hinaus ist es besonders bevorzugt, dass der Strom 6 in den Verfahrensschritt (a) der Hydrocyanierung zumindest teilweise zurückgeführt wird. Dabei ist es möglich, dass der zurückgeführte Katalysator teilweise einer Regeneration, beispielsweise wie in der deutschen Patentanmeldung DE ... mit dem Titel, "Einsatz von azeotropgetrocknetem Nickel(II)-halogenid" der BASF AG (B03/0484) beschrieben, unterzogen wird.

Der Gehalt an 2-Methyl-3-butennitril in diesem zurückgeführten Strom 6 beträgt in einer bevorzugten Ausführungsform des erfindungsgemäßen Verfahrens weniger als 10 Gew.-%, besonders bevorzugt weniger als 5 Gew.-%, insbesondere weniger als 1 Gew.-%. Dieses wird dadurch erreicht, dass zwischen der Abzugsstelle für Strom 5 und der Abzugsstelle für Strom 6 genügend destillative Trennstufen vorgesehen werden.

Die thermische Belastung des Katalysators kann in einer bevorzugten Ausführungsform dadurch niedrig gehalten werden, dass die Sumpftemperatur 140 °C nicht überschreitet, was durch geeignete Druckverhältnisse sichergestellt werden kann.

Zudem ist es auch möglich, den Strom 6 aus Verfahrensschritt (c) ganz oder teilweise als Katalysatorstrom für andere Hydrocyanierungen zu verwenden, beispielsweise zur Hydrocyanierung von 3-Pentennitril. Auch wenn der Katalysatorstrom 6 zur Hydrocyanierung von 3-Pentennitril verwendet wird, ist es bevorzugt, dass der Gehalt an 2-Methyl-3-butennitril in diesem Katalysatorstrom 6 möglichst gering ist und die zuvor genannten Werte nicht übersteigt.

In einer weiteren bevorzugten Ausführungsform wird ein Frischkatalysatorstrom in die Destillationsvorrichtung von Verfahrensschritt (c) gefahren, um den Pentennitril-Gehalt des gesamten Katalysatorstroms zu Verfahrensschritt (a) in den oben angegebenen Grenzen kontrollieren zu können.

In einer weiteren bevorzugten Ausführungsform des erfindungsgemäßen Verfahrens wird die Menge der Katalysatorausschleusung und damit die nötige Ergänzungsmenge an Frischkatalysator so bemessen, dass im Katalysatorkreislauf der Gehalt an Methlyglutardinitril nicht über 50 Gew.-%, besonders bevorzugt nicht über 20 Gew.-%, insbesondere nicht über 10 Gew.-%, jeweils bezogen auf den Katalysatorkreislaufstrom, steigt, um den jeweils ausgeschleusten Katalysatorstrom in einer Regenerierung mit möglichst wenig hemmenden Effekten von Methylglutardinitril zur Aufnahme von Nickel(0) vorliegen zu haben.

In einer weiteren bevorzugten Ausführungsform des erfindungsgemäßen Verfahrens wird die Menge der Katalysatorausschleusung und damit die nötige Ergänzungsmenge an Frischkatalysator so bemessen, dass im Katalysatorkreislauf der Gehalt an Nickel(0)-Komplexen nicht unter 0,05 Gew.-% fällt, besonders bevorzugt nicht unter 0,1 Gew.-%, insbesondere nicht unter 0,2 Gew.-%, jeweils bezogen auf den Katalysatorkreislauf und jeweils berechnet als metallisches Nickel(0), um die Aktivität des Hydrocyanierungskatalysators trotz Verlusten von Nickel(0)-Komplexen während der Reaktion in Schritt (a) oder während der Destillationsverfahren in Schritt (b) und (c), insbesondere während der Reaktion in Schritt (a), sicherzustellen.

Der Strom 5 wird anschließend in ***Verfahrensschritt (d)*** in eine weitere Destillationsvorrichtung überführt. In dieser Destillationsvorrichtung erfolgt eine Destillation des Stromes 5 unter Erhalt eines Stromes 7, der 2-Methyl-3-butennitril enthält, und eines Stromes 8, der 3-Pentennitril enthält. Der Strom 7 wird am Kopf der Destillationsvorrichtung erhalten, während der Strom 8 im Sumpf der Destillationsvorrichtung erhalten wird.

Dabei wird in einer besonders bevorzugten Ausführungsform des erfindungsgemäßen Verfahrens der gegebenenfalls als gasförmiger Seitenabzug erhaltene Strom 5 gasförmig in die Destillationsvorrichtung von Verfahrensschritt (d) überführt, wobei der Druck an der Position der Zulaufstelle für Strom 5 in der Destillationsvorrichtung von Verfahrensschritt (d) kleiner oder gleich dem Druck an der Position des Seitenabzugs für Strom 5 in der Destillationsvorrichtung von Verfahrensschritt (c) ist.

Nicht ausgenommen vom Umfang dieser Beschreibung sind Verfahrensvarianten, bei denen der Druck der Stufe (d) frei gewählt wird und der Gasstrom 5 gegebenenfalls auf einen höheren Druck als an der Entnahmestelle in (c) verdichtet oder durch Kondensation verflüssigt und gegebenenfalls mit einer Pumpe gefördert wird, um der Stufe (d) zugeführt werden zu können.

Der Verfahrensschritt (d) des erfindungsgemäßen Verfahrens kann in jeder geeigneten, dem Fachmann bekannten Apparatur durchgeführt werden. Für diese Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen oder einstufige Verdampfer, wie Fallfilmverdampfer, Dünnschichtverdampfer, Flashverdampfer, Mehrphasenwendelrohrverdampfer, Naturumlaufverdampfer oder Zwangsumlaufentspannungsverdampfer. Die Destillation kann in mehreren, wie zwei oder drei Apparaturen, vorzugsweise in einer einzigen Apparatur durchgeführt werden.

Die Kolonnen enthalten vorzugsweise strukturierte Packungen. Dabei erzeugen die strukturierten Packungen vorzugsweise 5 bis 100, besonders bevorzugt 10 bis 80, insbesondere 15 bis 50, theoretische Trennstufen.

Der Druck in Verfahrensschritt (d) beträgt vorzugsweise 0,001 bis 100 bar, besonders bevorzugt 0,01 bis 20 bar, insbesondere 0,05 bis 2 bar. Die Destillation wird so durchgeführt, dass die Temperatur im Sumpf der Destillationsvorrichtung vorzugsweise 30 bis 250 °C, besonders bevorzugt 50 bis 200 °C, insbesondere 60 bis 180 °C, beträgt. Die Destillation wird so durchgeführt, dass die Kondensationstemperatur am Kopf der Destillationsvorrichtung vorzugsweise -50 bis 250 °C, besonders bevorzugt 0 bis 180 °C, insbesondere 15 bis 160 °C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten
In einer Ausführungsform des erfindungsgemäßen Verfahrens kann Strom 7, der in dem Verfahrensschritt (d) erhalten wird, einer Isomersierung gemäß der DE-A 102 004 004 671 zugeführt werden.

In einer Ausführungsform des erfindungsgemäßen Verfahrens kann Strom 7, der in dem Verfahrensschritt (d) erhalten wird, in den Verfahrensschritt (a) und/oder in Verfahrensschritt (b) zurückgeführt werden, wobei die Reaktionsbedingungen in Verfahrensschritt (a) oder die Verweilzeit der flüssigen Phase im Sumpf von Verfahrensschritt (b) so gewählt werden, dass 2-Methyl-3-butennitril zumindest teilweise zu trans-3-Pentennitril isomerisiert wird.

In einer weiteren Ausführungsform des erfindungsgemäßen Verfahrens wird der Strom 7 als Seitenabzugsstrom in der Destillationsvorrichtung des Verfahrensschritts (d) gewonnen, wobei als Kopfprodukt dieser Destillationskolonne ein Strom erhalten wird, der neben 2-Methyl-3-butennitril im Wesentlichem noch (Z)-2-Methyl-2-butennitril und gegebenenfalls 1,3-Butadien und Buten-Isomere sowie Vinylcyclohexen und Ethylidencyclohexen enthält. Diese Ausführungsform ist vorteilhaft, da der Strom 7 dann reicher an 2-Methyl-3-butennitril ist als der Kopfstrom.

Der Gehalt an trans-3-Pentennitril in dem Strom 7 beträgt vorzugsweise 0 bis 50 Gew.-%, besonders bevorzugt 100 Gew.-ppm bis 20 Gew.-%, insbesondere 1 bis 15 Gew.-%. Der Gehalt an 2-Methyl-3-butennitril in dem Strom 8 beträgt vorzugsweise 0 bis 10 Gew.-%, besonders bevorzugt 5 Gew.-ppm bis 5 Gew.-%, insbesondere 50 Gew.-ppm bis 1 Gew.-%.

Das erfindungsgemäße Verfahren ermöglicht die Herstellung von 3-Pentennitril und 2-Methyl-3-butennitril in einem integrierten Verfahren, das aufgrund der nahezu vollständig möglichen Rückführung der 1,3-Butadienströme und des Katalysatorstroms eine hohe Verfahrensausbeute für die Einsatzstoffe aufweist. Dabei sind die zur destillativen Abtrennung von 1,3-Butadien und Pentennitril-Isomeren aus den katalysatorhaltigen Strömen nötigen Temperaturen und Druckverhältnisse so wählbar, dass einerseits die Sumpfverdampfertemperaturen bei Ausübung des Verfahrens im Produktionsmaßstab mit technisch erreichbaren Verweilzeiten so niedrig sind, dass sie vorzugsweise nicht zu einer Katalysatorschädigung führen und dass andererseits die Kondensation der Kopfprodukte der jeweiligen Destillationsschritte vorzugsweise bei Temperaturen stattfinden, bei denen die Wärmeabfuhr im Produktionsmaßstab mit wirtschaftlich vertretbarem Aufwand möglich ist.

Die vorliegende Erfindung wird anhand der nachfolgenden Ausführungsbeispiele näher erläutert.

In den Beispielen werden folgende Abkürzungen verwendet:
- BD:: 1,3-Butadien
- TBC:: tert-Butylbrenzkatechin
- C2BU:: cis-2-Buten
- T3PN:: trans-3-Pentennitril
- 2M3BN:: 2-Methyl-3-butennitril
- Z2M2BN:: (Z)-2-Methyl-2-butennitril
- E2M2BN:: [E]-2-Methyl-2-butennitril
- MGN:: Methylglutardinitril und
- ADN:: Adipodinitril
- HCN:: Cyanwasserstoff
- KAT:: Katalysator
- REG:: Regenerierung

### Beispiel 1:

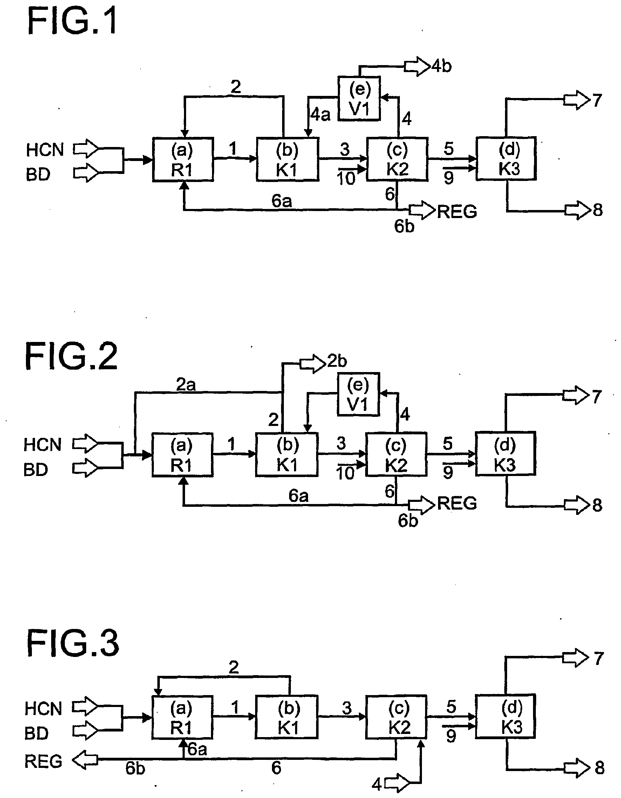

Beispiel 1 wird anhand Figur 1 verdeutlicht.

In Beispiel 1 wird für die Hydrocyanierung von BD ein Katalysatorsystem auf Basis von Nickel(0)-Komplexen mit einem Gemisch von Liganden eingesetzt. Die Ligandmischung zur Hydrocyanierung enthält ca. 60 Mol Gew.-% Tri(m/p-tolyl)phosphit und 40 Mol Gew.-% des Chelatphosphonits 1:

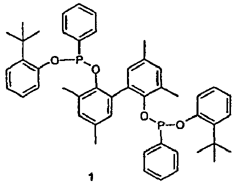

In einem Verfahrensschritt (a) werden folgende Ströme in einen Schlaufenreaktor R1 von 25 I Volumen gefahren, der mit einer Düse, Impulsaustauschrohr, externen Umpumpkreislauf und im einem im Umpumpkreislauf befindlichen Wärmetauscher zur Abfuhr der Reaktionsenergie ausgestattet ist und auf 357 K temperiert ist:
(1) 10 kg/h flüssiger, unstabilisierter, durch Destillation von Wasser befreiter Cyanwasserstoff,
(2) 22 kg/h handelsübliches BD, enthaltend 0,25 Gew.-% C2BU, das durch Kontakt mit Aluminiumoxid behandelt wurde, um Wasser und Stabilisator TBC zu entfernen,
(3) 8 kg/h rückgeführtes BD aus K1 in Verfahrensschritt (b) (Strom 2), so dass als gesamter BD-Zulauf zum Reaktor R1 ein Strom von 30 kg/h enthaltend 90 Gew.-% BD, 5 Gew.-% C2BU sowie 5 Gew.-% 1-Buten erhalten wird,
(4) 21 kg/h Nickel(0)-Katalysatorlösung, erhalten wie in diesem Beispiel weiter unten beschrieben als Strom 6a aus der Kolonne K2.

Der aus dem Reaktor R1 abgezogene Strom 1 (63 kg/h) enthält in Summe 11 Gew.-% BD und C2BU, entsprechend einem Umsatz von 79 % BD, sowie in Summe 63 Gew.-% Pentennitrile, 31 Gew.-% T3PN, 29 Gew.% 2M3BN, untergeordnete Mengen cis-3-Pentennitril, trans-2-Pentennitril, cis-2-Pentennitril, 4-Pentennitril und geringe Mengen Z2M2BN und E2M2BN, sowie die Katalysatorbestandteile und Katalysatorabbauprodukte und MGN.

Strom 1 wird in einem Verfahrensschritt (b) einer Destillationskolonne K1 zugeführt, die mit Verstärkungs- und Abtriebsteil betrieben wird und mit einem Fallfilmverdampfer und einem getrennten Sumpf ausgestattet ist, sowie Kolonneneinbauten mit strukturierter Packung enthält, die 10 theoretische Trennstufen erzeugen. Kolonne K1 wird am Kopf mit einem Direktkondensator betrieben der aus einem mit strukturierter Packung bestückten Kolonnenschuss mit Totalfangtasse, Umpumpkreis und externen Wärmetauscher besteht. Die Kolonne K1 wird bei einem absoluten Druck von 2,0 bar Kopfdruck, 288 K Kopftemperatur und 363 K Sumpfabzugstemperatur betrieben.

Über Kopf der Kolonne K1 wird der Strom 2 erhalten, der wie eingangs beschrieben als Rückführstrom in den Reaktor R1 dosiert wird. Das Rücklaufverhältnis am Kopf der Kolonne K1 wird so eingestellt, dass der Strom 2 ca. 100 ppm 2M3BN enthält.

Über Sumpf der Kolonne K1 werden 59 kg/h eines Stromes 3 erhalten, der 2,9 Gew.-% BD, 4,6 Gew.-% C2BU, 67 Gew.-% Pentennitrile sowie zusätzlich die Katalysatorbestandteile enthält. C2BU ist im Verhältnis zu BD gegenüber dem Zulauf deutlich angereichert.

Strom 3 wird in einem Verfahrensschritt (c) in eine Destillationskolonne K2 gefahren, die in Abtriebsfahrweise betrieben wird und mit Fallfilmverdampfer, Kopfkondensator mit Nachkondensator sowie Kolonneneinbauten mit strukturierter Packung ausgestattet ist, die 10 theoretische Trennstufen erzeugen. Die Kolonne wird bei einem absoluten Druck von 150 mbar Kopfdruck, 329 K Kopftemperatur und 373 K Sumpfabzugstemperatur betrieben. Der Brüdenstrom der Kolonne wird bei 308 K teilkondensiert und mit einem Nachkondensator bei 263 K behandelt. Der so von 2M3BN und anderen Pentennitrilen abgereicherte Strom 4 wird in einem Verdichter V1 auf einen absoluten Druck von 1,2 bar verdichtet. Der verdichtete Gasstrom wird bei 279 K zum großen Teil unter Erhalt eines Stromes 4a (5 kg/h) kondensiert, wobei ein Teilstrom 4b (ca. 50 NI/h, enthaltend 44 Gew.-% C2BU) gasförmig entsorgt wird. Strom 4a wird flüssig in den Rücklaufteil des geteilten Sumpfes der Kolonne K1 zurückgefahren.

An der Kolonne K2 wird in einem gasförmigen Seitenabzug der Strom 5 gewonnen (40 kg/h), enthaltend ca. 50 ppm BD, 46 Gew.-% 2M3BN und 48 Gew.-% T3PN sowie in geringerem Umfang E2M2BN und Z2M2BN neben anderen Pentennitril-Isomeren. Die Position des Seitenabzugs ist so gewählt, dass unter dem Seitenabzug in einem Abtriebsteil die Komponente 2M3BN in dem über Sumpf gewonnen Strom 6 im Verhältnis zu T3PN abgereichert wird.

In die Kolonne K2 werden 13 kg/h eines Katalysatorstromes 10 gefahren, enthaltend in Summe 73 Gew.-% Pentennitrile, 0,5 Gew.% Ni(0), 18 Gew.-% Ligandmischung sowie ca. 5 Gew.-% ADN.

An der Kolonne K2 wird über Sumpf der Katalysator-Strom 6 erhalten, enthaltend 0,5 Gew.-% Ni(0), ca. 100 ppm 2M3BN und 35 Gew.-% restliche Pentennitrile. Der Strom 6 wird teilweise in den Reaktor R1 zurückgefahren (Strom 6a) (21 kg/h). Ein anderer Teil (Strom 6b) (5,4 kg/h) wird einer Regenerierung (REG), beispielsweise beschrieben in der DE-A-103 51 002 zugeführt, um nach Regenerierung beispielsweise in Beispiel 1 der Hydrocyanierung von 3-Pentennitril gemäß der DE-A-102 004 004 683 eingesetzt zu werden.

Der Strom 5 wird in einem Verfahrensschritt (d) zu einer Destillationskolonne K3 gefahren, die mit Umlaufverdampfer und Kopfkondensator sowie mit strukturierter Packung ausgestattet ist, die 30 theoretische Trennstufen erzeugen. Die Kolonne K3 wird bei einem absoluten Druck 180 mbar Kopfdruck, 345 K Kopftemperatur und 363 K Sumpfabzugstemperatur betrieben.

In die Kolonne K3 werden 39 kg/h eines Stromes 9 gefahren, enthaltend 54 Gew.-% T3PN, 23 Gew.-% 2M3BN und 16 Gew.-% Z2M2BN sowie in geringen Mengen weitere Pentennitril-Isomere. Strom 9 kann beispielsweise als rückgeführter Pentennitrilstrom aus einem Verfahren zur Isomerisierung von 2-Methyl-3-butennitril zu 3-Pentennitril erhalten werden, wie in Beispiel 1 der DE-A-102 004 004 671 beschrieben.

Über Kopf der Kolonne K3 werden 40 kg/h eines Stromes 7 erhalten, enthaltend 10 Gew.-% T3PN, 68 Gew.-% 2M3BN, 16 Gew.-% Z2M2BN sowie in Summe 0,1 Gew.% BD und C2BU. Dieser Strom kann einem Verfahren zur Isomerisierung von 2-Methyl-3-butennitril zu 3-Pentennitril zugeführt werden, wie in Beispiel 1 der deutschen Patentanmeldung DE ... mit dem Titel "Verfahren zur Herstellung von linearem Pentennitril" der BASF AG (B03/0436) beschrieben.

Über Sumpf der Kolonne K3 werden 39 kg/h des Stromes 8 erhalten, enthaltend in Summe 97 Gew.-% T3PN, C3PN und 4PN sowie ca. 100 ppm 2M3BN und ca. 1 Gew.-% E2M2BN.

Beispiel 1 zeigt, wie eine nahezu vollständige Rückgewinnung von 1,3-Butadien in einem Hydrocyanierungsverfahren gelingt. In Beispiel 1 wird die Aufpegelung von cis-2-Buten im Butadienkreislauf einerseits mit dem Betrieb von Kolonne K1 mit einem Abtriebsteil und andererseits durch die Ausschleusung eines purge-Stromes 4b am Verdichter V1 erreicht, wobei der Strom 4b (ca. 50 Nl/h) ca. 40 Vol.-% cis-2-Buten enthält.

Der in Beispiel 1 vorgefundene Verlust an 1,3-Butadien ist klein gegenüber dem Beispiel 2, in dem die Kolonne K1 ohne Abtriebsteil betrieben wird und der zur Begrenzung der Aufpegelungen nötige Purge-Strom als Strom 2b am Kopf der Kolonne K1 abgezogen wird (330 NI/h) (mit nur 7 Gew.-% cis-2-Buten und 92 Gew.-% 1,3-Butadien, das wirtschaftlich bedeutsamen Verlusten zuzurechnen ist).

### Beispiel 2: (Referenz)

Beispiel 2 wird anhand Figur 2 verdeutlicht.

In Beispiel 2 wird für die Hydrocyanierung von BD ein Katalysatorsystem auf Basis von Nickel(0)-Komplexen mit Chelatphosphonit 1 als Ligand verwendet:

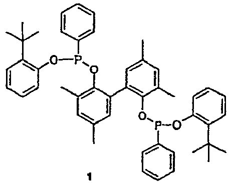

In einem Verfahrensschritt (a) werden folgende Ströme in einen Schlaufenreaktor R1 von 25 I Volumen gefahren, der mit einer Düse, Impulsaustauschrohr, externen Umpumpkreislauf und einem im Umpumpkreislauf befindlichen Wärmetauscher zur Abfuhr der Reaktionsenergie ausgestattet ist und auf 357 K temperiert ist:
(1) 10 kg/h flüssiger, unstabilisierter, durch Destillation von Wasser befreiter Cyanwasserstoff,
(2) 22 kg/h handelsübliches BD, enthaltend 0,25 Gew.-% C2BU, das durch Kontakt mit Molsieb behandlet wurde, um Wasser auf Konzentrationen kleiner 10 ppm zu entfernen,
(3) 8 kg/h rückgeführtes BD aus K1 in Verfahrensschritt (b) (Strom 2a), so dass als gesamter BD-Zulauf zum Reaktor R1 ein Strom von 30 kg/h enthaltend 90 Gew.-% BD, 8 Gew.-% C2BU sowie 2 Gew.-% 1-Buten erhalten wird,
(4) 21 kg/h Nickel(0)-Katalysatorlösung, erhalten wie in diesem Beispiel weiter unten beschrieben als Strom 6a aus der Kolonne K2.

Der aus dem Reaktor R1 abgezogene Strom 1 (63 kg/h) enthält in Summe 13 Gew.-% BD und C2BU, entsprechend einem Umsatz von 79 % BD, sowie in Summe 63 Gew.-% Pentennitrile, 31 Gew.-% T3PN, 29 Gew.-% 2M3BN, untergeordnete Mengen cis-3-Pentennitril, trans-2-Pentennitril, cis-2-Pentennitril, 4-Pentennitril und geringe Mengen Z2M2BN und E2M2BN, sowie die Katalysatorbestandteile und Katalysatorabbauprodukte und MGN.

Strom 1 wird in einem Verfahrensschritt (b) einer Destillationskolonne K1 zugeführt, die mit Verstärkungsteil betrieben wird und mit einem Fallfilmverdampfer und getrennten Sumpf ausgestattet ist, sowie Kolonneneinbauten enthält, die 2 theoretische Trennstufen erzeugen. Kolonne K1 wird am Kopf mit einem Direktkondensator betriebenen der aus einem mit Füllkörperschüttung bestückten Kolonnenschuss mit Totalfangtasse, Umpumpkreis und externen Wärmetauscher besteht. Die Kolonne K1 wird bei einem absoluten Druck von 2,0 bar Kopfdruck, 290 K Kopftemperatur und 363 K Sumpfabzugstemperatur betrieben.

Aus dem Kondensatorkreislaufstrom am Kopf der Kolonne K1 wird der Strom 2 erhalten, der wie eingangs beschrieben teilweise als Rückführstrom 2a in den Reaktor R1 dosiert wird. Das Rücklaufverhältnis am Kopf der Kolonne K1 wird so eingestellt, dass der Strom 2 ca. 100 ppm 2M3BN enthält.

Als gasförmiger Strom wird aus dem Kopfkondensator der Kolonne K1 ein Ausschleusstrom Strom 2b entnommen (ca. 330 NI/h), enthaltend 92 Gew.-% Butadien und 7 Gew.-% cis-2-Buten sowie geringe Mengen 1-Buten. Die Ausschleusstrommenge ist so bemessen, dass im Butadienrückführstrom 2a in Summe ca. 10 Gew.-% 2-Butenisomere und 1-Buten enthalten sind.

Über Sumpf der Kolonne K1 werden 59 kg/h eines Stromes 3 erhalten, der 4,1 Gew.-% BD, 3,9 Gew.-% C2BU, 67 Gew.-% Pentennitrile sowie zusätzlich die Katalysatorbestandteile enthält.

Strom 3 wird in einem Verfahrensschritt (c) in eine Destillationskolonne K2 gefahren, die in Abtriebsfahrweise betrieben wird und mit Fallfilmverdampfer, Kopfkondensator mit Nachkondensator sowie Kolonneneinbauten mit strukturierter Packung ausgestattet ist, die 10 theoretische Trennstufen erzeugen. Die Kolonne wird bei einem absoluten Druck von 150 mbar Kopfdruck, 354 K Kopftemperatur und 371 K Sumpfabzugstemperatur betrieben.

Der Brüdenstrom der Kolonne wird bei 288 K teilkondensiert und mit einem Nachkondensator bei 263 K behandelt. Der so von 2M3BN und anderen Pentennitrilen abgereicherte gasförmige Strom 4 (5 kg/h), enthaltend 46 Gew.-% Butadien, 45 Gew.-% cis-2-Buten und ca. 5 Gew.-% Pentennitrilisomere, wird in einem Verdichter V1 auf einen absoluten Druck von mehr als 2,0 bar dergestalt verdichtet, dass die auf der Druckseite des Verdichters erreichte Druckdifferenz zur Kolonne K1 ausreicht, um den verdichteten Gasstrom gasförmig in die Kolonne K1 zurückfahren zu können.

An der Kolonne K2 wird in einem gasförmigen Seitenabzug der Strom 5 gewonnen (40 kg/h), enthaltend ca. 50 ppm BD, 46 Gew.-% 2M3BN und 48 Gew.-% T3PN sowie in geringerem Umfang E2M2BN und Z2M2BN neben anderen Pentennitril-Isomeren. Die Position des Seitenabzugs ist so gewählt, dass unter dem Seitenabzug in einem Abtriebsteil die Komponente 2M3BN in dem über Sumpf gewonnen Strom 6 im Verhältnis zu T3PN abgereichert wird.

In die Kolonne K2 werden 13 kg/h eines Katalysatorstromes 10 gefahren, enthaltend in Summe 73 Gew.-% Pentennitrile, 0,5 Gew.-% Ni(0), 18 Gew.-% Ligandmischung sowie ca. 5 Gew.-% ADN.

An der Kolonne K2 wird über Sumpf der Katalysator-Strom 6 erhalten (27 kg/h), enthaltend 1,0 Gew.-% Ni(0), ca. 2000 ppm 2M3BN und in Summe 35 Gew.-% restliche Pentennitrile. Der Strom 6 wird teilweise (Strom 6a) in den Reaktor R1 zurückgefahren (21 kg/h). Ein anderer Teil (Strom 6b) (5,4 kg/h) kann einer Regenerierung (REG), beispielsweise beschrieben in der DE-A-103 51 002, zugeführt werden.

Der Strom 5 wird in einem Verfahrensschritt (d) zu einer Destillationskolonne K3 gefahren, die mit Umlaufverdampfer und Kopfkondensator sowie mit strukturierter Packung ausgestattet ist, die 30 theoretische Trennstufen erzeugen. Die Kolonne K3 wird bei einem absoluten Druck von 180 mbar Kopfdruck, 345 K Kopftemperatur und 363 K Sumpfabzugstemperatur betrieben.

In die Kolonne K3 werden 39 kg/h eines Stromes 9 gefahren, enthaltend 54 Gew.-% T3PN, 23 Gew.-% 2M3BN und 16 Gew.-% Z2M2BN sowie in geringen Mengen weitere Pentennitril-Isomere. Strom 9 kann beispielsweise als rückgeführter Pentennitrilstrom aus einem Verfahren zur Isomerisierung von 2-Methyl-3-butennitril zu 3-Pentennitril erhalten werden, wie in Beispiel 1 der DE-A-102 004 004 671 beschrieben.

Über Kopf der Kolonne K3 werden 40 kg/h eines Strom 7 erhalten, enthaltend 10 Gew.-% T3PN, 68 Gew.-% 2M3BN, 16 Gew.-% Z2M2BN sowie in Summe 0,1 Gew.-% BD und C2BU. Dieser Strom kann einem Verfahren zur Isomerisierung von 2-Methyl-3-butennitril zu 3-Pentennitril zugeführt werden, wie in Beispiel 1 der DE-A-102 004 004 671 beschrieben.

Über Sumpf der Kolonne K3 werden 39 kg/h des Stromes 8 erhalten, enthaltend in Summe 97 Gew.-% T3PN, C3PN und 4PN sowie ca. 100 ppm 2M3BN und ca. 1 Gew.-% E2M2BN.

In Beispiel 3 kann gezeigt werden, dass bei einer Beispiel 2 ähnlichen Fahrweise deutlich weniger REG-Verluste in Strom 2b hingenommen werden müssen, wenn die Kolonne K1 mit einem Abtriebsteil ausgestattet ist, da über die Kolonne K2 im Wesentlichen cis-2-Buten an Stelle 1,3-Butadien zur Kolonne K3 ausgeschleust wird.

### Beispiel 3: (Referenz)

Beispiel 3 wird ebenfalls anhand Figur 2 verdeutlicht.

In Beispiel 3 wird für die Hydrocyanierung von Butadien ein Katalysatorsystem auf Basis von Nickel(0)-Komplexen mit Chelatphosphonit 1 als Ligand:

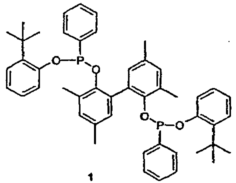

In einem Verfahrensschritt (a) werden folgende Ströme in einen Schlaufenreaktor R1 von 25 l Volumen gefahren, der mit einer Düse, Impulsaustauschrohr, externen Umpumpkreislauf und im einem im Umpumpkreislauf befindlichen Wärmetauscher zur Abfuhr der Reaktionsenergie ausgestattet ist und auf 357 K temperiert ist:
(1) 10 kg/h flüssiger, unstabilisierter, durch Destillation von Wasser befreiter Cyanwasserstoff,
(2) 22 kg/h handelsübliches BD, enthaltend 0,25 Gew.-% C2BU, das durch Kontakt mit Molsieb behandelt wurde, um Wasser auf Konzentrationen kleiner 10 ppm zu entfernen,
(3) 8 kg/h rückgeführtes BD aus K1 in Verfahrensschritt (b) (Strom 2a), so dass als gesamter BD-Zulauf zum Reaktor R1 ein Strom von 30 kg/h enthaltend 90 Gew.-% BD, 4 Gew.-% C2BU sowie 6 Gew.-% 1-Buten erhalten wird,
(4) 21 kg/h Nickel(0)-Katalysatorlösung, erhalten wie in diesem Beispiel weiter unten beschrieben als Strom 6a aus der Kolonne K2.

Der aus dem Reaktor R1 abgezogene Strom 1 (63 kg/h) enthält in Summe 13 Gew.-% BD und C2BU, entsprechend einem Umsatz von 79 % BD, sowie in Summe 63 Gew.-% Pentennitrile, 31 Gew.-% T3PN, 29 Gew.-% 2M3BN, untergeordnete Mengen cis-3-Pentennitril, trans-2-Pentennitril, cis-2-Pentennitril, 4-Pentennitril und geringe Mengen Z2M2BN und E2M2BN, sowie die Katalysatorbestandteile und Katalysatorabbauprodukte und MGN.

Strom 1 wird in einem Verfahrensschritt (b) einer Destillationskolonne K1 zugeführt, die mit Verstärkungs- und Abtriebsteil betrieben wird und mit einem Fallfilmverdampfer und einem getrennten Sumpf ausgestattet ist, sowie Kolonneneinbauten mit strukturierter Packung enthält, die 10 theoretische Trennstufen erzeugen. Kolonne K1 wird am Kopf mit einem Direktkondensator betrieben der aus einem mit strukturierter Packung bestückten Kolonnenschuss mit Totalfangtasse, Umpumpkreis und externen Wärmetauscher besteht. Die Kolonne K1 wird bei einem absoluten Druck von 2,0 bar Kopfdruck, 288 K Kopftemperatur und 363 K Sumpfabzugstemperatur betrieben.

Aus dem Kondensatorkreislaufstrom am Kopf der Kolonne K1 wird der Strom 2 erhalten, der wie eingangs beschrieben teilweise als Rückführstrom 2a in den Reaktor R1 dosiert wird. Das Rücklaufverhältnis am Kopf der Kolonne K1 wird so eingestellt, dass der Strom 2 ca. 100 ppm 2M3BN enthält.

Als gasförmiger Strom wird aus dem Kopfkondensator der Kolonne K1 ein Ausschleusstrom Strom 2b entnommen (ca. 55 NI/h), enthaltend 93 Gew.-% Butadien und 3 Gew.-% cis-2-Buten sowie geringe Mengen 1-Buten. Die Ausschleusstrommenge ist so bemessen, dass im Butadienrückführstrom 2a in Summe ca. 10 Gew.-% 2-Butene und 1-Buten enthalten sind.

Über Sumpf der Kolonne K1 werden 59 kg/h eines Stromes 3 erhalten, der 2,2 Gew.-% BD, 6,3 Gew.-% C2BU, 67 Gew.-% Pentennitrile sowie zusätzlich die Katalysatorbestandteile enthält.

Strom 3 wird in einem Verfahrensschritt (c) in eine Destillationskolonne K2 gefahren, die in Abtriebsfahrweise betrieben wird und mit Fallfilmverdampfer, Kopfkondensator mit Nachkondensator sowie Kolonneneinbauten mit strukturierter Packung ausgestattet ist, die 10 theoretische Trennstufen erzeugen. Die Kolonne wird bei einem absoluten Druck von 150 mbar Kopfdruck, 354 K Kopftemperatur und 371 K Sumpfabzugstemperatur betrieben.

Der Brüdenstrom der Kolonne wird bei 313 K teilkondensiert und mit einem Nachkondensator bei 263 K behandelt. Der so von 2M3BN und anderen Pentennitrilen abgereicherte Strom 4 (5 kg/h), enthaltend in 23 Gew.-% Butadien, 66 Gew.-% cis-2-Buten und ca. 5 Gew.-% Pentennitrilisomere wird in einem Verdichter V1 auf einen absoluten Druck von mehr als 2,0 bar dergestalt verdichtet, dass die auf der Druckseite des Verdichters erreichte Druckdifferenz zur Kolonne K1 ausreicht, um den verdichteten Gasstrom gasförmig in die Kolonne K1 zurückfahren zu können.

An der Kolonne K2 wird in einem gasförmigen Seitenabzug der Strom 5 gewonnen (40 kg/h), enthaltend ca. 200 ppm BD, 46 Gew.-% 2M3BN und 48 Gew.-% T3PN sowie in geringerem Umfang E2M2BN und Z2M2BN neben anderen Pentennitril-Isomeren. Die Position des Seitenabzugs ist so gewählt, dass unter dem Seitenabzug in einem Abtriebsteil die Komponente 2M3BN in dem über Sumpf gewonnen Strom 6 im Verhältnis zu T3PN abgereichert wird.

In die Kolonne K2 werden 13 kg/h eines Katalysatorstromes 10 gefahren, enthaltend in Summe 73 Gew.-% Pentennitrile, 0,5 Gew.-% Ni(0), 18 Gew.-% Ligandmischung sowie ca. 5 Gew.-% ADN.

An der Kolonne K2 wird über Sumpf der Katalysator-Strom 6 erhalten, enthaltend 1,0 Gew.-% Ni(0), ca. 2000 ppm 2M3BN und in Summe 35 Gew.-% restliche Pentennitrile. Der Strom 6 wird teilweise (Strom 6a) in den Reaktor R1 zurückgefahren (21 kg/h). Ein anderer Teil (Strom 6b) (5,4 kg/h) kann einer Regenerierung (REG), beispielsweise beschrieben in der DE-A-1 03 51 002, zugeführt werden.

Der Strom 5 wird in einem Verfahrensschritt (d) zu einer Destillationskolonne K3 gefahren, die mit Umlaufverdampfer und Kopfkondensator sowie mit strukturierter Packung ausgestattet ist, die 30 theoretische Trennstufen erzeugen. Die Kolonne K3 wird bei einem absoluten Druck von 180 mbar Kopfdruck, 345 K Kopftemperatur und 363 K Sumpfabzugstemperatur betrieben.

In die Kolonne K3 werden 39 kg/h eines Stromes 9 gefahren, enthaltend 54 Gew.-% T3PN, 23 Gew.-% 2M3BN und 16 Gew.-% Z2M2BN sowie in geringen Mengen weitere Pentennitril-Isomere. Strom 9 kann beispielsweise als rückgeführter Pentennitrilstrom aus einem Verfahren zur Isomerisierung von 2-Methyl-3-butennitril zu 3-Pentennitril erhalten werden, wie in Beispiel 1 der DE-A-102 004 004 671 beschrieben.

Über Kopf der Kolonne K3 werden 40 kg/h eines Stromes 7 erhalten, enthaltend 10 Gew.-% T3PN, 68 Gew.-% 2M3BN, 16 Gew.-% Z2M2BN sowie ca. 0,1 Gew.-% BD und ca. 1,5 Gew.-% C2BU. Dieser Strom kann einem Verfahren zur Isomerisierung von 2-Methyl-3-butennitril zu 3-Pentennitril zugeführt werden, wie in Beispiel 1 der DE-A-102 004 004 beschrieben.

Über Sumpf der Kolonne K3 werden 39 kg/h des Stromes 8 erhalten, enthaltend in Summe 97 Gew.-% T3PN, C3PN und 4PN sowie ca. 100 ppm 2M3BN und ca. 1 Gew.-% E2M2BN. Strom 8 kann einem Verfahren zur Hydrocyanierung von 3-Pentennitril zu Adipodinitril zugeführt werden, wie in Beispiel 1 der Hydrocyanierung von 3-Pentennitril gemäß der DE-A-102 004 004 683 beschrieben.

### Vergleichsbeispiel:

Das Vergleichsbeispiel wird anhand Figur 3 verdeutlicht.

Im Vergleichsbeispiel wird für die Hydrocyanierung von 1,3-Butadien ein Katalysatorsystem auf Basis von Nickel(0)-Komplexen mit Chelatphosphit 2 als Ligand verwendet:

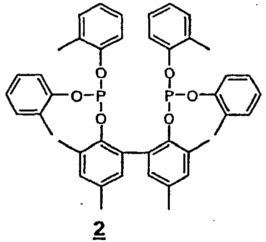

In einem Verfahrensschritt (a) werden folgende Ströme in ein System aus zwei Reaktoren R1a und R1b von je 12 I Volumen gefahren, die jeweils mit einer Düse, Impulsaustauschrohr, externen Umpumpkreislauf und im einem im Umpumpkreislauf befindlichen Wärmetauscher zur Abfuhr der Reaktionsenergie ausgestattet sind und auf 363 K temperiert sind:
(1) 6 kg/h flüssiger, unstabilisierter, durch Destillation von Wasser befreiter Cyanwasserstoff zu R1a,
(2) 6 kg/h flüssiger, unstabilisierter, durch Destillation von Wasser befreiter Cyanwasserstoff zu R1b,
(3) 25 kg/h handelsübliches BD zu R1a, enthaltend 0,25 Gew.-% C2BU, das durch Kontakt mit Aluminiumoxid behandelt wurde, um Wasser und Stabilisator TBC zu entfernen,
(4) 2 kg/h rückgeführtes BD aus Kolonne K1 in Verfahrensschritt (b) zu R1a (Strom 2), so dass als gesamter BD-Zulauf zum Reaktor R1 ein Strom von 27 kg/h enthaltend 98 Gew.-% BD und in Summe 2 Gew.-% C2BU und 1-Buten erhalten wird,
(5) 14 kg/h Nickel(0)-Katalysatorlösung zu R1a, erhalten wie in diesem Beispiel weiter unten beschrieben als Strom 6a aus der Kolonne K2.

Der aus dem Reaktor R1b abgezogene Strom 1 (54 kg/h) enthält in Summe 4 Gew.-% BD und C2BU, entsprechend einem Umsatz von 94 % BD, sowie in Summe 74 Gew.% Pentennitrile, davon 33 Gew.% T3PN, 37 Gew.% 2M3BN, untergeordnete Mengen cis-3-Pentennitril, trans-2-Pentennitril, cis-2-Pentennitril, 4-Pentennitril und geringe Mengen Z2M2BN und E2M2BN, sowie die Katalysatorbestandteile und Katalysatorabbauprodukte und MGN.

Strom 1 wird in einem Verfahrensschritt 2 in einer Destillationskolonne K1 zugeführt, die als Verstärkungskolonne betrieben wird und mit einem Fallfilmverdampfer ausgestattet ist, sowie Kolonneneinbauten mit strukturierter Packung enthält, die 4 theoretische Trennstufen erzeugen. Kolonne K1 wird am Kopf mit einem Direktkondensator betriebenen der aus einem mit Füllkörperschüttung bestückten Kolonnenschuss mit Totalfangtasse, Umpumpkreis und externen Wärmetauscher besteht. Die Kolonne K1 wird bei einem absoluten Druck von 0,8 bar Kopfdruck, 263 K Kopftemperatur und 393 K Sumpfabzugstemperatur betrieben.

Über Kopf der Kolonne K1 wird der Strom 2 erhalten, der wie eingangs beschrieben als Rückführstrom in den Reaktor R1a dosiert wird. Das Rücklaufverhältnis am Kopf der Kolonne K1 wird so eingestellt, dass der Strom 2 0,1 Gew.-% 2M3BN enthält.

Über Sumpf der Kolonne K1 werden 52 kg/h eines Stromes 3 erhalten, der 0,3 Gew.-% BD, 0,1 Gew.-% C2BU, 76 Gew.-% Pentennitrile sowie zusätzlich die Katalysatorbestandteile enthält.

Strom 3 wird innerhalb des Verfahrensschrittes (c) in eine Destillationskolonne K2 gefahren, die in Abtriebsfahrweise betrieben wird und mit Fallfilmverdampfer, Kopfkondensator mit Nachkondensator sowie Kolonneneinbauten mit strukturierter Packung ausgestattet ist, die 4 theoretische Trennstufen erzeugen. Die Kolonne wird bei einem absoluten Druck von 70 mbar Kopfdruck, 333 K Kopftemperatur und 373 K Sumpfabzugstemperatur betrieben.

An der Kolonne K2 wird der gasförmige Kopfabzug Strom 5 gewonnen (40 kg/h), enthaltend 0,4 Gew.-% BD, 54 Gew.-% 2M3BN und 42 Gew.-% T3PN sowie in geringerem Umfang E2M2BN und Z2M2BN neben anderen Pentennitril-Isomeren.
In die Kolonne K2 werden 3 kg/h eines Katalysatorstromes 4 gefahren, enthaltend in Summe 45 Gew.-% Pentennitrile, 1,5 Gew.-% Ni(0) und den Chelatligand, erhalten beispielsweise durch Umsetzung von Nickel(0)(Cyclooctadienyl)₂-Komplex mit dem Chelatphosphit 2.

An der Kolonne K2 wird über Sumpf der Katalysator-Strom 6 erhalten, enthaltend 1,2 Gew.-% Ni(0), 0,3 Gew.-% 2M3BN und 17 Gew.-% restliche Pentennitrile. Der Strom 6 wird teilweise (Strom 6a) in den Reaktor R1 zurückgefahren (14 kg/h). Ein anderer Teil (Strom 6b) (3,8 kg/h) wird einer Regenerierung (REG), beispielsweise beschrieben in der DE-A-103 51 002, zugeführt, und kann nach der Regenerierung beispielsweise in der Hydrocyanierung von 3-Pentennitril eingesetzt oder gegebenenfalls in die Hydrocyanierung von Butadien laut dem erfindungsgemäßem Verfahren zurückgeführt werden.

Der Strom 5 wird in einem Verfahrensschritt (d) zu einer Destillationskolonne K3 gefahren, die mit Umlaufverdampfer und Kopfkondensator sowie mit strukturierter Packung ausgestattet ist, die 45 theoretische Trennstufen erzeugen. Die Kolonne K3 wird bei einem absoluten Druck von 1,0 bar Kopfdruck, 395 K Kopftemperatur und 416 K Sumpfabzugstemperatur betrieben.

In die Kolonne K3 werden 24 kg/h (Strom 9) zugefahren, enthaltend 70 Gew.-% T3PN, 14 Gew.-% 2M3BN und 7 Gew.-% Z2M2BN sowie in geringen Mengen weitere Pentennitril-Isomere. Strom 9 kann beispielsweise als rückgeführter Pentennitrilstrom aus einem Verfahren zur Isomerisierung von 2-Methyl-3-butennitril zu 3-Pentennitril erhalten werden, wie in Beispiel 2 der DE-A-102 004 004 671 beschrieben.

Über Kopf der Kolonne K3 werden 30 kg/h eines Stromes 7 erhalten, enthaltend 1 Gew.-% T3PN, 85 Gew.-% 2M3BN, 8 Gew.-% Z2M2BN sowie in Summe 3 Gew.-% BD und C2BU. Das Rücklaufverhältnis der Kolonne K3 wird so eingestellt, dass über Kopf 1 Gew.-% 3PN erhalten werden. Dieser Strom kann einem Verfahren zur Isomerisierung von 2-Methyl-3-butennitril zu 3-Pentennitril erhalten werden, wie in Beispiel 2 der DE-A-102 004 004 671 beschrieben.

Über Sumpf der Kolonne K3 werden 38 kg/h des Stromes 8 erhalten, enthaltend in Summe 97 Gew.-% T3PN, C3PN und 4PN sowie ca. 10 ppm 2M3BN und ca. 2 Gew.-% E2M2BN und in geringen Mengen MGN. Strom 8 kann einem Verfahren zur Hydrocyanierung von 3-Pentennitril zu Adipodinitril zugeführt werden, wie in Beispiel 2 der Hydrocyanierung von 3-Pentennitril gemäß der DE-A-102 004 004 683 .

Das Vergleichsbeispiel zeigt, dass sowohl ohne die zweistufige Butadien-Abtrennung in den Destillationsstufen K1 und K2 mit Rückführung des 1,3-Butadiens ohne Rückverdichtung oder ohne den Betrieb der Destillationsstufe K1 als Abtriebskolonne deutlich ungünstigere Temperatur- und Druckverhältnisse in der Stufe K1 anzuwenden sind, um eine 1,3-Butadien-Verlustrate zu erzielen, die den Werten in Beispiel 1 bis 3 nahe kommt. Die dann nötigen Temperaturen zur ausreichend vollständigen 1,3-Butadien-Rückführung in der Kolonne K1 (120 °C im Vergleichsbeispiel an Stelle 90 °C in den Beispielen 1 bis 3) führen bei den temperaturempfindlichen Chelatliganden und den Nickelkomplexen, unabhängig ob Phosphit oder Phosphonite verwendet werden, zu Katalyatorverlusten. Der bei 120 °C Sumpftemperatur für Abreicherung auf ca. 0,5 Gew.-% 1,3-Butadien nötige Druck von ca. 0,8 bar führt zu sehr niedrigen Temperaturen von -10 °C am Kopfkondensator, um 1,3-Butadien kondensieren und flüssig in die Reaktoren zurückführen zu können. Die Abfuhr von Kondensationswärme auf diesem Temperaturniveau des Vergleichsbeispiels ist wirtschaftlich ungleich aufwendiger als beispielsweise mit Kühlwasser, wie in Beispiel 1 möglich.

## Patentansprüche

1. Verfahren zur Herstellung von 3-Pentennitril durch Hydrocyanierung von 1,3-Butadien, **gekennzeichnet durch** die folgenden Verfahrensschritte:
(a) Umsetzung von 1,3-Butadien, das cis-2-Buten enthält, mit Cyanwasserstoff an mindestens einem Katalysator unter Erhalt eines Stromes 1, der 3-Pentennitril, 2-Methyl-3-butennitril, den mindestens einen Katalysator, 1,3-Butadien und Reste von noch nicht umgesetztem Cyanwasserstoff enthält,
(b) Destillation des Stromes 1 in einer Destillationsvorrichtung K1 unter Erhalt eines Stromes 2 als Kopfprodukt, der den überwiegenden Teil des 1,3-Butadiens aus Strom 1 enthält, und eines Stromes 3 als Sumpfprodukt, der 3-Pentennitril, den mindestens einen Katalysator, 2-Methyl-3-butennitril und den restlichen Teil des 1,3-Butadiens aus Strom 1 enthält, der nicht in Strom 2 abgetrennt wurde,
(c) Destillation des Stromes 3 in einer Destillationsvorrichtung K2 unter Erhalt eines Stromes 4 als Kopfprodukt, der 1,3-Butadien enthält, eines Stromes 5 an einem Seitenabzug der Kolonne, der 3-Pentennitril und 2-Methyl-3- butennitril enthält, und eines Stromes 6 als Sumpfprodukt, der den mindestens einen Katalysator enthält,
(d) Destillation des Stromes 5 unter Erhalt eines Stromes 7 als Kopfprodukt, der 2-Methyl-3-butennitril enthält, und eines Stromes 8 als Sumpfprodukt, der 3-Pentennitril enthält, wobei
die in Verfahrensschritt (b) verwendete Destillationsvorrichtung K1 mindestens eine Destillationskolonne mit einem Abtriebsteil umfasst und gegebenenfalls
die in Verfahrensschritt (c) verwendete Destillationsvorrichtung K2 zwischen dem Zulauf des Stromes 3 und dem Abzug des Stromes 5 destillative Trennstufen aufweist und der Abzug des Stromes 5 in der Destillationsvorrichtung K2 tiefer als der Zulauf von Strom 3 angeordnet ist, und wobei
der in Verfahrensschritt (b) erhaltene Strom 2, der 1,3-Butadien enthält, in Verfahrensschritt (a) und der in Verfahrensschritt (c) erhaltene Strom 4, der 1,3-Butadien enthält, in Verfahrensschritt (a) und/oder (b) zurückgefahren werden, und
ein Teilstrom 4b aus dem in Verfahrensschritt (c) erhaltenen Strom 4 ausgeschleust wird.

2. Verfahren nach Anspruch 1, **dadurch gekennzeichnet, dass** die in Verfahrensschritt (b) verwendete Destillationskolonne K1 2 bis 60 theoretische Trennstufen aufweist.

3. Verfahren nach einem der Ansprüche 1 oder 2, **dadurch gekennzeichnet, dass** die in Verfahrensschritt (b) verwendete Destillationsvorrichtung K1 unterhalb des Zulaufs von Strom 1 Trennstufen aufweist, die eine Anreicherung von cis-2-Buten gegenüber 1,3-Butadien in Strom 3 ermöglichen und ein Teilstrom 4b aus dem in Verfahrensschritt (c) erhaltenen Strom 4 ausgeschleust wird.

4. Verfahren nach einem Ansprüche 1 bis 3, **dadurch gekennzeichnet, dass** die Ausschleusung gasförmig erfolgt.

5. Verfahren nach einem der Ansprüche 1 oder 2, **dadurch gekennzeichnet, dass** im Verstärkungsteil der Destillationskolonne K1 in Verfahrensschritt (b) ein Strom an einem Seitenabzug der Destillationsvorrichtung K1 im Siedezustand erhalten, an einem Kondensator durch indirekte Wärmeabfuhr unter Erhalt eines unterkühlten Stromes kondensiert und auf den Kopf der Destillationsvorrichtung K1 des Verfahrensschrittes (b) zurückgeführt wird, wobei vor oder nach der Kondensation ein Strom 2'abgezogen wird und der Strom 2' anstelle des Stromes 2 in Verfahrensschritt (a) zurückgeführt wird.

6. Verfahren nach einem der Ansprüche 1 bis 5, **dadurch gekennzeichnet, dass** in Verfahrensschritt (c) vor Erhalt von Strom 4 nitrilhaltige Verbindungen aus dem Brüdenstrom durch mehrstufige Kondensation abgereichert werden.

7. Verfahren nach einem der Ansprüche 1 bis 6, **dadurch gekennzeichnet, dass** dem Verfahrensschritt (a) zusätzlich zu dem zurückgeführten 1,3-Butadien benötigtes 1,3-Butadien zugeführt wird.

8. Verfahren nach einem der Ansprüche 1 bis 7, **dadurch gekennzeichnet, dass** im Verfahren verwendetes 1,3-Butadien keinen Stabilisator aufweist, wobei durch eine geeignete Wahl der Druckverhältnisse die Kondensationstemperaturen im Kopfbereich der Destillationseinrichtung K1 von Verfahrensschritt (b) kleiner 293 K gehalten werden, um eine Polymerisation von 1,3-Butadien zu verhindern, insbesondere um das Wachstum von Popcorn-Polymerkeimen zu begrenzen.

## Claims

1. A process for preparing 3-pentenenitrile by hydrocyanating 1,3-butadiene, **characterized by** the following process steps:
(a) reacting 1,3-butadiene which comprises cis-2-butene with hydrogen cyanide over at least one catalyst to obtain a stream 1 which comprises 3-pentenenitrile, 2-methyl-3-butenenitrile, the at least one catalyst, 1,3-butadiene and residues of hydrogen cyanide which has yet to be converted,
(b) distilling stream 1 in a distillation apparatus K1 to obtain a stream 2 as the top product which comprises the predominant portion of the 1,3-butadiene from stream 1, and a stream 3 as the bottom product which comprises 3-pentenenitrile, the at least one catalyst, 2-methyl-3-butenenitrile and the remaining portion of the 1,3-butadiene from stream 1 which has not been removed in stream 2,
(c) distilling stream 3 in a distillation apparatus K2 to obtain a stream 4 as the top product which comprises 1,3-butadiene, a stream 5 which comprises 3-pentenenitrile and 2-methyl-3-butenenitrile at a side draw of the column, and a stream 6 as the bottom product which comprises the at least one catalyst,
(d) distilling stream 5 to obtain a stream 7 as the top product which comprises 2-methyl-3-butenenitrile, and a stream 8 as the bottom product which comprises 3-pentenenitrile, wherein
the distillation apparatus K1 used in process step (b) comprises at least one distillation column having a stripping section and optionally
the distillation apparatus K2 used in process step (c) has distillative separation stages between the feed of stream 3 and the draw of stream 5, and the draw of stream 5 is disposed lower in the distillation apparatus K2 than the feed of stream 3, and wherein
the stream 2 which is obtained in process step (b) and comprises 1,3-butadiene is recycled into process step (a), and the stream 4 which is obtained in process step (c) and comprises 1,3-butadiene is recycled into process step (a) and/or (b), and
a substream 4b from the stream 4 obtained in process step (c) is discharged.

2. The process according to claim 1, wherein the distillation column K1 used in process step (b) has from 2 to 60 theoretical plates.

3. The process according to either of claims 1 and 2, wherein the distillation apparatus K1 used in process step (b) has separation stages below the feed of stream 1 which enable enrichment of cis-2-butene relative to 1,3-butadiene in stream 3, and a substream 4b from the stream 4 obtained in process step (c) is discharged.

4. The process according to any of claims 1 to 3, wherein the discharge is in gaseous form.

5. The process according to either of claims 1 and 2, wherein, in the rectifying section of the distillation column K1 in process step (b), a stream is obtained in the boiling state at a side draw of the distillation apparatus K1, condensed on a condenser by indirect heat removal to obtain a cooled stream and recycled to the top of the distillation apparatus K1 of process step (b), and a stream 2' is drawn off before or after the condensation and the stream 2' is recycled into process step (a) instead of stream 2.

6. The process according to any of claims 1 to 5, wherein, in process step (c) before stream 4 is obtained, nitrile-containing compounds are depleted from the vapor stream by multistage condensation.

7. The process according to any of claims 1 to 6, wherein 1,3-butadiene required in addition to the recycled 1,3-butadiene is fed to process step (a).

8. The process according to any of claims 1 to 7, wherein 1,3-butadiene used in the process has no stabilizer, and a suitable selection of the pressure conditions keeps the condensation temperatures in the top region of the distillation apparatus K1 of process step (b) less than 293 K in order to prevent polymerization of 1,3-butadiene, especially in order to limit the growth of popcorn polymer nuclei.

## Revendications

1. Procédé de fabrication de 3-pentène-nitrile par hydrocyanation de 1,3-butadiène, **caractérisé par** les étapes de procédé suivantes :
(a) la mise en réaction de 1,3-butadiène, qui contient du cis-2-butène, avec du cyanure d'hydrogène sur au moins un catalyseur pour obtenir un courant 1, qui contient du 3-pentène-nitrile, du 2-méthyl-3-butène-nitrile, le ou les catalyseurs, du 1,3-butadiène et des résidus de cyanure d'hydrogène encore non réagi,
(b) la distillation du courant 1 dans un dispositif de distillation K1 pour obtenir un courant 2 en tant que produit de tête, qui contient la majeure partie du 1,3-butadiène du courant 1, et un courant 3 en tant que produit de fond, qui contient du 3-pentène-nitrile, le ou les catalyseurs, du 2-méthyl-3-butène-nitrile et la partie résiduelle du 1,3-butadiène du courant 1, qui n'a pas été séparée dans le courant 2,
(c) la distillation du courant 3 dans un dispositif de distillation K2 pour obtenir un courant 4 en tant que produit de tête, qui contient du 1,3-butadiène, un courant 5 au niveau d'une sortie latérale de la colonne, qui contient du 3-pentène-nitrile et du 2-méthyl-3-butène-nitrile, et un courant 6 en tant que produit de fond, qui contient le ou les catalyseurs,
(d) la distillation du courant 5 pour obtenir un courant 7 en tant que produit de tête, qui contient du 2-méthyl-3-butène-nitrile, et un courant 8 en tant que produit de fond, qui contient du 3-pentène-nitrile,
le dispositif de distillation K1 utilisé lors de l'étape de procédé (b) comprenant au moins une colonne de distillation munie d'une zone de rectification et éventuellement le dispositif de distillation K2 utilisé lors de l'étape de procédé (c) comprenant des étapes de séparation par distillation entre l'alimentation du courant 3 et le soutirage du courant 5 et le soutirage du courant 5 dans le dispositif de distillation K2 étant placé plus bas que l'alimentation du courant 3, et le courant 2 obtenu lors de l'étape de procédé (b), qui contient du 1,3-butadiène, étant recyclé dans l'étape de procédé (a) et le courant 4 obtenu lors de l'étape de procédé (c), qui contient du 1,3-butadiène, dans l'étape de procédé (a) et/ou (b), et un courant partiel 4b étant déchargé à partir du courant 4 obtenu lors de l'étape de procédé (c).

2. Procédé selon la revendication 1, **caractérisé en ce que** la colonne de distillation K1 utilisée lors de l'étape de procédé (b) comprend 2 à 60 étapes de séparation théoriques.

3. Procédé selon l'une quelconque des revendications 1 et 2, **caractérisé en ce que** le dispositif de distillation K1 utilisé lors de l'étape de procédé (b) comporte des étapes de séparation sous l'alimentation du courant 1, qui permettent un enrichissement du cis-2-butène par rapport au 1,3-butadiène dans le courant 3 et un courant partiel 4b est déchargé à partir du courant 4 obtenu lors de l'étape de procédé (c).

4. Procédé selon l'une quelconque revendications 1 à 3, **caractérisé en ce que** le déchargement a lieu sous forme gazeuse.

5. Procédé selon l'une quelconque des revendications 1 et 2, **caractérisé en ce qu'**un courant est obtenu à l'état d'ébullition au niveau d'une sortie latérale du dispositif de distillation K1 dans la zone d'enrichissement de la colonne de distillation K1 lors de l'étape de procédé (b), condensé dans un condensateur par dissipation indirecte de chaleur pour obtenir un courant sous-refroidi et recyclé à la tête du dispositif de distillation K1 de l'étape de procédé (b), un courant 2' étant soutiré avant ou après la condensation et le courant 2' étant recyclé dans l'étape de procédé (a) au lieu du courant 2.

6. Procédé selon l'une quelconque des revendications 1 à 5, **caractérisé en ce que** des composés contenant du nitrile sont appauvris dans le courant de vapeurs par condensation à plusieurs étapes lors de l'étape de procédé (c) avant l'obtention du courant 4.

7. Procédé selon l'une quelconque des revendications 1 à 6, **caractérisé en ce que** du 1,3-butadiène nécessaire est introduit dans l'étape de procédé (a) en plus du 1,3-butadiène recyclé.

8. Procédé selon l'une quelconque des revendications 1 à 7, **caractérisé en ce que** le 1,3-butadiène utilisé dans le procédé ne comprend pas de stabilisateur, les températures de condensation dans la zone de tête du dispositif de distillation K1 de l'étape de procédé (b) étant maintenues inférieures à 293 K par un choix approprié des rapports de pression afin d'éviter une polymérisation du 1,3-butadiène, notamment afin de limiter la croissance de germes de polymères popcorn.
